# EUROPÄISCHE PATENTSCHRIFT

(11) **EP 2 217 287 B1**
(45) Veröffentlichungstag und Bekanntmachung des Hinweises auf die Patenterteilung: **20.07.2011**
(21) Anmeldenummer: 08848174.2
(22) Anmeldetag: 10.11.2008
(51) Int. Cl.: A61L 15/32, A61L 15/60

(54) **HYGIENE- ODER PFLEGEARTIKEL, AUFWEISEND EINEN ANTEIL AN HYDROAKTIVEN POLYMEREN, UND EINE ZUBEREITUNG AUFWEISEND BAKTERIOPHAGEN ODER MINDESTENS EINEN BESTANDTEIL DERSELBEN**
HYGIENE OR CARE ITEM COMPRISING A MOIETY OF HYDRO-ACTIVE POLYMERS, AND PREPARATION CONTAINING BACTERIOPHAGES OR AT LEAST ONE COMPONENT THEREOF
ARTICLE HYGIÉNIQUE OU DE SOINS CONTENANT UNE QUANTITÉ DE POLYMÈRES HYDROACTIFS, ET PRÉPARATION PRÉSENTANT DES BACTÉRIOPHAGES OU AU MOINS UN CONSTITUANT DE CES BACTÉRIOPHAGES

(30) Priorität: 11.11.2007 DE 102007054127
(43) Veröffentlichungstag der Anmeldung: 18.08.2010
(73) Patentinhaber: Riesinger, Birgit, 48346 Ostbevern (DE)
(72) Erfinder: Riesinger, Birgit, 48346 Ostbevern (DE)
(74) Vertreter: Michalski Hüttermann & Partner Patentanwälte
(86) Internationale Anmeldenummer: PCT/EP2008/065237
(87) Internationale Veröffentlichungsnummer: WO 2009/060097

(56) Entgegenhaltungen:
- EP-A- 1 435 247
- WO-A-2006/047870
- US-A1- 2002 001 590

## Beschreibung

Die Erfindung betrifft einen Hygiene- oder Pflegeartikel gemäß dem Oberbegriff des Anspruchs 1.

Ein solcher Hygiene- oder Pflegeartikel eignet sich insbesondere zur Aufnahme von Exsudat aus chronischen Wunden, wie z.B. bei Diabetes, Ulcus cruris und ähnlichen Erkrankungen auftreten.

Ebenso eignet sich ein solcher Hygiene- oder Pflegeartikel auch für die Aufnahme von anderen Flüssigkeiten, insbesondere von Körperflüssigkeiten und -ausscheidungen.

Der Begriff "Exsudat" bezeichnet eine über die entzündlichen Prozesse des Wundödems vom Blutplasma abgeleitete Wundflüssigkeit. So wie das Blut für den Transport von Nährstoffen und anderen Botenstoffen und damit für die Versorgung verschiedener Teile des Körpers verantwortlich ist, dient das Exsudat auf ganz ähnliche Weise der Versorgung des Wundbettes und der darin ablaufenden Heilungsprozesse. Um dieser Vielzahl an Funktionen gerecht zu werden, enthält es ein breites Spektrum an Komponenten, woraus ein spezifisches Gewicht resultiert, das leicht oberhalb dessen von Wasser liegt. Darin unterscheidet es sich auch vom Transsudat, welches von nicht-entzündlichen Prozessen abgeleitet ist und ein deutlich geringeres spezifisches Gewicht mit einem geringen Zell- und Proteingehalt aufweist. Neben der Bereitstellung von Nährstoffen für die Fibroblasten und Epithelzellen koordiniert das Exsudat die verschiedenen Prozesse der Wundheilung zeitlich und räumlich durch seinen hohen Gehalt an Wachstumsfaktoren und Zytokinen. Diese werden vor allem durch Thrombozyten, Keratinozyten, Makrophagen und Fibroblasten gebildet. Sie beeinflussen die Motilität, Migration und Proliferation der verschiedenen an der Wundheilung beteiligten Zellen. So wird das Einwandern von Zellen in den Wundgrund ebenso gefördert wie die Versorgung des neugebildeten Granulationsgewebes durch die Angiogenese. Auch die Wundreinigung wird durch das Exsudat unterstützt. Es enthält verschiedene Serin-, Cystein- und Aspartatproteasen sowie Matrix-Metalloproteasen, die in ihrer Aktivität streng reguliert irreversibel geschädigtes Gewebe abbauen und somit das Wundbett für die nachfolgenden Phasen der Heilung vorbereiten.

Bestandteile des physiologischen Exsudats sind insbesondere Salze, Glucose, Zytokine und Wachstumsfaktoren, Plasmaproteine, Proteasen (insbesondere Matrix-Metalloproteasen), Granulozyten und Makrophagen.

Kommt es nicht innerhalb einiger Wochen zu einer deutlichen Progression des Wundheilungsverlaufs entsprechend der verschiedenen Phasen der Wundheilung, so spricht man von einer chronischen Wunde. Dabei betrachtet man jedoch bereits länger als drei Tage andauernde exsudative Phasen als Komplikation und spricht von einer pathologischen Exsudation, welche zu einer Chronifizierung der Wunde beitragen kann. Die zugrunde liegenden Ursachen sind meist komplex und können durchaus auch systemischer Natur sein. Es überrascht jedoch aufgrund der zuvor erläuterten Bedeutung des Exsudats für die Wundheilung nicht, dass sich Komplikationen der Wundheilung in einer deutlich veränderten Zusammensetzung und Wirkung des Exsudats widerspiegeln.

Unter anderem durch eine Konzentrationsverschiebung der einzelnen Bestandteile des Exsudats verliert das normalerweise heilungsfördernde Exsudat bei chronischen Wunden seine positive Wirkung. Insbesondere der Gehalt an inflammatorischen Zytokinen und Proteasen ist in pathologischem Exsudat signifikant erhöht. Der Gehalt an Wachstumsfaktoren ist dagegen verringert. Ein besonders gravierender Unterschied ergibt sich hinsichtlich der Aktivität der zuvo rangesprochenen Matrix-Metalloproteasen. Neben der Vorbereitung des Wundbetts sind sie auch beim späteren Umbau des Granulations- zum Narbengewebe beteiligt. Diese Enzyme werden normalerweise als inaktives Präenzym gebildet und in ihrer Aktivierung durch entsprechende Inhibitoren reguliert (tissue inhibitors of metalloproteases, TIMPs), welche gleichzeitig selbst eine positive Wirkung auf das Zellwachstum haben. Im chronischen Exsudat scheint aufgrund von Störungen in diesem Regulationssystem die Aktivität der Proteasen erhöht, was möglicherweise zu einer aktiven Wundregression beiträgt. Das pathologische Exsudat ist hinsichtlich des Gehalts seiner Komponenten aus dem der Wundprogressionförderlichen Gleichgewicht geraten. Daraus ergeben sich verschiedene Komplikationen, die zur weiteren Verschlechterung und Chronifizierung der Wunde beitragen.

Insbesondere bei chronischen, schlecht heilenden Wunden, wie sie z.B. bei Diabetikern häufig anzutreffen sind, spielen Infektionen eine große Rolle. Dieses Problem hat sich in der jüngeren Vergangenheit durch antibiotikaresistente pathogene Keime verschärft. Insbesondere multiresistente Keime wie z.B. Vancomycin-resistente Enterokokken, Methicillin-Resistente Staphylococcus aureus und multiresistente Pseudomonas aeruginosa spielen in der klinischen Praxis heute eine oftmals fatale Rolle.

So werden in den USA jährlich bei 90.000 Ulcus-Cruris-Patienten mit Diabetes Gliedmaßen amputiert, da diese aufgrund infektiöser Komplikationen durch multiresistente Keime nicht anders behandelbar sind.

Bei Inkontinenzartikeln, insbesondere bei Windeln, besteht hingegen die Gefahr, das sich insbesondere Vancomycin-resistente Enterokokken, die in einem solchen Artikel, wenn dieser benutzt wird, ideale Lebensbedingungen vorfinden, vermehren und z.B. wunde Hautstellen der den Artikel tragenden Person befallen. Dies ist insbesondere deswegen eine reelle Gefahr, da Inkontinenzpatienten häufig auch bettlägerig sind und z.B. einen Dekubitus aufweisen (beispielsweise im Steißbereich), bei welchem sich bei Einwanderung solcher resistenter Keime eine äußerst schwer zu behandelnde Infektion ausbilden könnte. Überdies ist bei solchen Patienten aufgrund ihrer mitunter langen Krankengeschichte die Gefahr der Existenz Vancomycin-resistenter Enterokokken im Verdauungstrakt gegeben.

Aber auch bei anderen Hygiene- und/oder Wundpflegeartikeln wie z.B. Monatsbinden, Tampons, Inkontinenzartikeln, Windeln, Krankenunterlagen, Stomabeuteln, Stomabeuteleinlagen, Fußmatten, OP-Tüchern, Redonflaschen, Taschentüchern und/oder Hyperhidrose-Artikeln besteht die Gefahr, dass sich antibiotikaresistente pathogene Keime aufgrund der oftmals günstigen Wachstumsbedingungen in denselben vermehren und eine Infektionsgefahr darstellen.

Die Verwendung von Bakteriophagen zur Bekämpfung bakterieller Infektionen wird seit den zwanziger Jahren des letzten Jahrhunderts diskutiert.

Als Bakteriophagen bezeichnet man eine Gruppe von Viren, die auf Bakterien als Wirtszellen spezialisiert sind. Bakteriophagen werden taxonomisch nach ihrer Morphologie, ihrem Genom und ihrem Wirt eingeteilt. So unterscheidet man DNA-Phagen mit einzel- oder doppelsträngiger DNA bzw RNA (ss-DNA-Phagen, ds-DNA-Phagen, ss-RNA-Phagen bzw. dsRNA-Phagen).

Der Aufbau eines Pohagen soll hier kurz am Beispiel der Phagen der T-Reihe skizziert werden. Diese Phagen setzen sich aus einer Grundplatte, einem Injektionsapparat und einem Kopf, dem so genannten Kapsid zusammen. Die Grundplatte (die wie Kapsid und Injektionsapparat aus Proteinen aufgebaut ist) ist mit Schwanzfibern und Spikes besetzt, die der Adsorption auf der Bakterienwand dienen. Der Injektionsapparat besteht aus einem dünnen Rohr, auch Schwanzrohr genannt, durch das die Phagen-DNA in das Wirtsbakterium injiziert wird. Das Rohr wird von einer kontraktilen Schwanzscheide umhüllt, die sich während der Injektion zusammenzieht. Das Kapsid ist aus 152 Kapsomeren zu einer ikosaedrischen Symmetrie aufgebaut und enthält die DNA des Phagen.

Bakteriophagen benötigen wie alle Viren mangels eines eigenen Stoffwechsels zur Reproduktion einen Wirt, im Falle der Bakteriophagen eine lebende (geeignete) Bakterienzelle. Die Reproduktion lässt sich in fünf Phasen untergliedern:
1. Adsorption an spezifische Rezeptoren der Zellwand des Wirtsbakteriums
2. Injektion der Phagen-DNA in das Wirtsbakterium
3. Transkription des Virusgenoms, Translation der viralen mRNA durch den bakteriellen Transkriptionsapparat, Replikation der Virusnukleinsäure und Entstehung von Virusbauteilen,
4. Zusammenbau ("self assembly") zu reifen Phagenpartikeln
5. Freisetzung der fertigen Viruspartikel durch Lyse des Wirtsbakteriums

Aufgrund einiger Rückschläge und angesichts des überwältigenden Erfolgs der zeitgleich eingeführten Antibiotika ließ das Interesse an phagentherapeutischen Verfahren in der Mitte des letzten Jahrhunderts nach, und die Verfahren gerieten in Vergessenheit. In einigen Ländern Osteuropas, insbesondere Russland und Georgien, wurden phagentherapeutische Verfahren jedoch weiter entwickelt, was nicht zuletzt auf das Bestreben nach Unabhängigkeit von der westlichen Pharmaindustrie zurückzuführen ist.

Mit dem Aufkommen der oben erwähnten multiresistenten Keime hat auch im Westen das Interesse an diesen Verfahren wieder zugenommen. So bietet z.B. die Firma Intralytix verscheidene Zubereitungen enthaltend Bakteriophagen für die Behandlung bakterieller Infektionserkrankungen beim Menschen an, so z.B. eine Zubereitung für die topische Anwendung (Markennname: WPP-201), die sich für die Behandlung von infektiösen Hautgeschwüren bei Diabetes-Patienten eignen soll. Das Produkt befindet sich kurz vor der klinischen Erprobung.

Ein anderes Beispiel ist ein auf Bakteriophagen basierendes antibakterielles Mittel (Markenname PhagoBioDerm), das auf einer biodegradierbaren Polymer-Matrix (bestehend aus Aminosäuren) besteht und mit natürlich vorkommenden Bakteriophagen imprägniert ist. Dieses Produkt kann verwendet werden, um bakterielle Infektionen in chronischen und akuten Wunden zu bekämpfen, und ist derzeit bereits in der Republik Georgien in Gebrauch (Jikia et al, Clin Exp Dermatol 30 (1), 23-26, 2005).

Die US2004146490 beschreibt ausgewählte Bakteriophagen und ihre Verwendung zur Abtötung bzw. Inhibition von Methicillin-resistenten *Staphylococcus aureus* (MRSA).

Allerdings weist der neue Ansatz einige Nachteile auf, die im folgenden aufgezählt werden sollen:

So macht die Wirtsspezifität von Bakteriophagen unterschiedliche Cocktails für die patienten- und/oder krankheitsspezifische Behandlung erforderlich, da die bakterielle Zusammensetzung patienten- und/oder krankheitsabhängig höchst variabel ist.

Für einige Erreger, wie z.B. Clostridium und Mykobakterium, sind bislang keine therapetischen Bakteriophagen bekannt.

Therapeutisch anzuwendende Bakteriophagen werden in der Regel auf einer Kultur enthaltend das pathogene Bakterium vermehrt und dann isoliert. Bei der Herstellung einer für therapeutische Zwecke vorgesehenen Zubereitung aufweisend Bakteriophagen oder Bestandteile derselben muß große Sorgfalt angewendet werden, um zu verhindern, dass die Zubereitung frei von bakteriellen Fragmenten und bakteriellen Endotoxinen ist.

Häufig bereitet auch der Transport der Bakteriophagen bzw. ihrer Bestandteile an den Wirkort erhebliche Probleme. Eine Injektion besagter Phagen würde zu einer Immunreaktion führen und die Phagen zerstören. Dies ist insbesondere bei systemischen Infektionen ein Problem, weniger bei topischen Infektionen insbesondere der Haut oder von Wunden.

Für einen maximalen Erfolg benötigt die Bakteriophagentherapie ein feuchtes Milieu mit hoher Luftfeuchtigkeit. Wird eine Bakteriophagenzubereitung als Salbe topisch verabreicht oder mit einem Zellstoffverband auf die Wunde aufgebracht, kann ein solches feuchtes Milieu häufig nicht gewährleistet werden.

Bei der Bakteriophagentherapie werden durch die lysierten Bakterien Endotoxine und bakterielle Pathogenitätsfaktoren (insbeondere bakterielles Hämolysin und Leukocidin) freigegeben, die beim Patienten Entzündungen, Allergien, Schocks (insbesondere anaphylaktische Schocks und/oder das Toxic Shock Syndome) und Fieber erzeugen können (Herxheimer-Reaktion). Dies gilt insbesondere dann, wenn eine Zubereitung aufweisend Bakteriophagen oder Bestandteile derselben topisch aufgebracht werden, z.B. in Form einer mit besagter Zubereitung ausgestatteten Wundauflage.

Insbesondere aus letzteren Gründen haben sich Wundauflagen mit Zubereitungen aufweisend Bakteriophagen oder deren Bestandteile bislang auf dem Markt nicht durchgesetzt.

Die DE10342104 beschreibt ein Verfahren zur Herstellung eines Mittels zur Wundbehandlung, bei welchem aus Eiern von Fliegen Maden gezüchtet werden, die in die zu behandelnde Wunde applizierbar sind. Dabei werden die Maden mit Bakteriophagen in der Weise kontaminiert werden, dass die applizierbaren Maden Träger der Bakteriophagen sind. Dieser Ansatz hat sich bislang in der Praxis nicht bewährt und löst eine Großteil der oben genannten Probleme nicht. Überdies weist er Akzeptanzprobleme bei zu behandelnden Patienten auf. Die DE10342071 beschreibt eine Vorrichtung zur Applikation von Bakteriophagen an eine Wundoberfläche, mit einer Einlage aus einem porösen Material zum Auflegen auf die Wundoberfläche, wobei eine Unterdruckquelle vorgesehen ist, mit deren Hilfe zerstörte Bakterien mit ihren Toxinen aus dem Gewebe abgesaugt werden. Eine solche Vorrichtung weist jedoch einen großen apparativen Aufwand auf (Unterdruckquelle, Vakuumabdeckung der Wunde, Absaugschlauch, Rückschlagventil, Auffangbehälter). Überdies muß die Vorrichtung kontrolliert und überwacht werden, insbesondere weil die Unterdruckquelle nur intervallartig eingeschaltet werden kann, und weist einen hohen Wartungsaufwand auf, der sie für den klinischen Alltag als ungeeignet erscheinen lässt.

WO 2006/047870 A1 offenbart einen Wundpflegeartikel, aufweisenden einen Anteil an hydroaktiven Polymeren, und eine Zubereitung aufweisend Bakteriophagen oder einen Bestandteil derselben.

US 2002/001590 A1 offenbart einen Wundpflegeartike, aufweisenden Bakteriophagen aus der Gruppe Myoviridae.

Aufgabe der vorliegenden Erfindung ist es, einen Hygiene- oder Pflegeartikel bereit zu stellen, der einige der oben genannten Nachteile nicht aufweist. Diese Aufgabe wird mit einem Hygiene- oder Pflegeartikel gemäß dem vorliegenden Hauptanspruch gelöst; die Unteranspruche geben bevorzugte Ausführungsformen an.

Demnach ist ein Hygiene- oder Pflegeartikel vorgesehen, aufweisend einen Anteil an hydroaktiven Polymeren, sowie eine Zubereitung aufweisend Bakteriophagen, oder mindestens einen Bestandteil derselben.

Bei der Zubereitung aufweisend Bakteriophagen kann es sich z.B. um eine wässrige Lösung, ein Spray, ein Aerosol, ein gefriergetrocknetes Produkt, ein kristallisiertes Produkt, eine Salbe, eine pastöse Zubereitung, ein Gel oder dergleichen handeln.

Bei besagtem Hygiene- oder Pflegeartikel handelt es sich bevorzugt um einen Artikel ausgewählt aus der Gruppe enthaltend Wundpflegeartikel, Monatsbinden, Tampons, Inkontinenzartikel, Windeln, Krankenunterlagen, Stomabeutel, Stomabeuteleinlagen, Fußmatten, OP-Tücher, Redonflaschen, Taschentücher und/oder Hyperhidrose-Artikel. Ebenso kann es sich auch um ein Pflegeprodukt handeln, so z.B. eine Salbe, eine pastöse Zubereitung, ein Gel oder dergleichen.

Der Begriff "Wundpflegeartikel" soll im Folgenden insbesondere eine Wundauflage, bevorzugt eine flächige Wundauflage oder ein Wundpflegetuch bezeichnen, die für den humanmedizinischen, dentalmedizinischen und/oder veterinärmedizinischen Sektor gedacht ist.

Besagter Wundpflegeartikel kann bevorzugt in Form eines Tupfers, eines Wundtuchs, einer Wundauflage, einer Wundkompresse, eines Wundpolsters, einer ggf. elastischen Wickel, einer Bandage, eines Pflasters oder eines Strumpfes vorliegen.

Insbesondere kann der Begriff "Wundpflegeartikel" auch als ein Ensemble verschiedener Produkte verstanden werden, die in einer gegebenen Anordnung auf der zu behandelnden Wunde angeordnet werden. Dieses Ensemble kann eine physikalische Einheit bilden, indem die verschiedenen Produkte in einer gemeinsamen Hülle zusammengefasst oder - ggf. ohne Hülle - adhäsiv miteinander verbunden sind. Das Ensemble kann jedoch auch in Form eines Kits vorliegen, bei dem die verschiedenen Produkte ggf. mithilfe einer Wickel, eines Klebebandes, eines Klebestreifens oder eines Pflasters in der gegebenen Anordnung auf der zu behandelnden Wunde angeordnet sind.

Es ist daher durch den Schutzbereich dieser Erfindung ausdrücklich auch ein mehrteiliger Hygiene- oder Pflegeartikel, aufweisend einen Anteil an hydroaktiven Polymeren, und eine Zubereitung aufweisend Bakteriophagen oder mindestens einen Bestandteil derselben, vorgesehen, wobei besagter Kit einen Hygiene- oder Pflegeartikel, insbesondere eine Wundauflage enthaltend hydroaktive Polymere sowie ein Produkt enthaltend Bakteriophagen aufweist.

Bei besagtem Hygiene- oder Pflegeartikel handelt es sich bevorzugt um eine Wundauflage enthaltend superabsorbierende Polymere, so z.B. das Produkt sorbion sachet" der Firma Sorbion, die Produkte "Tenderwet" und "Zetuvit" der Firma Hartmann, das Produkt "drymax" der Firma Absorbest oder ähnliche Produkte).

Bei besagtem Produkt enthaltend Bakteriophagen handelt es sich z.B. um das oben diskutierte Produkt "PhagoBioDerm" der Firma Intralytix, das auf einer biodegradierbaren Polymer-Matrix besteht und mit natürlich vorkommenden Bakteriophagen imprägniert ist).

Bevorzugt kann dabei vorgesehen sein, dass der Wundpflegeartikel als Spülkörper fungiert. Hiezu ist vorgesehen, dass der Wundpflegeartikel vor Aufbringen auf die Wunde mit einer physiologischen Lösung (z.B. 0.9 % Kochsalz, Ringerlösung oder dergleichen) getränkt wird oder entsprechend getränkt vorkonfektioniert vorliegt. Ein solcher Wundpflegeartikel gibt während der Verwendung kontinuierlich Flüssigkeit an die Wunde ab, spült diese und nimmt Exsudat, Zelltrümmer, nekrotische Bestandteile, Bakterien, Debris und dergleichen auf.

Unter dem Begriff "hydroaktive Polymere" sollen im folgenden Polymere verstanden werden, die feuchtigkeitsbindende Eigenschaften haben. Hierzu zählen u.a.
- Agar
- Alginat
- Carrageen
- Johannisbrotkernmehl
- Guarkernmehl
- Traganth
- Gummi arabicumd
- Xanthan
- Karaya
- Tarakernmehl
- Gellan
- Pektin
- Chitosan
- Hyaluronsäure
- Modifizierte Stärke
- Zellulose und Zelluloseether wie z.B. Carboxymethylcellulose, Hydroxypropylcellulose, Hydroxypropylmethylcellulose, Methylcellulose und Methylethylcellulose, sowie
- Superabsorbierende Polymere.

Die genannten hydroaktiven Polymere nehmen wässrige Wundexsudate auf und bilden dabei ebenfalls eine feuchte Oberfläche aus. Ggf. nehmen sie eine gelartige Form an. Die feuchte Oberfläche bzw. die Gelform trägt dazu bei, die Wundhaftneigung des Wundpflegeartikels zu reduzieren, so dass dieser sich nach Verwendung atraumatisch und schmerzlos ablösen lässt. Durch die Gelform weist der Wundpflegartikel eine kühlende und somit scherzlindernde Wirkung auf. Überdies ermöglicht die Gelform die Ausbildung eines wundheilungsfördernden Feuchtklimas.

Besonders bevorzugt handelt es sich bei den hydroaktiven Polymere um Alginate. Letztere werden aus den Braunalgen gewonnen und zu einem faserigen Vlies verwoben Chemisch handelt es sich um Polysaccharide, und zwar Calcium- und/oder Natrimsalze der Alginsäuren. Alginate können bis zum 20fachen ihres Eigengewichtes an Flüssigkeit aufnehmen, dabei wird das Wundexsudat in die Hohlräume eingelagert. Die im Alginatgitter enthaltenen Ca²⁺ Ionen werden gegen die Na⁺ Ionen aus dem Exsudat ausgetauscht, bis der Sättigungsgrad an Na-Ionen im Alginat erreicht ist. Dabei kommt es zu einem Aufquellen der Wundauflage und zur Umwandlung der Alginatfaser in einen Gelkörper durch Aufquellen der Fasern.

In einer anderen bevorzugten Ausführungsform handelt es sich bei den hydroaktiven Polymere um Carboxymethylcellulose. Letztere liegt insbesonderte in Form von Natriumcarboxymethylcellulose vor und ist z.B. unter dem Namen "Hydrofaser" im Handel. In Hygiene- und Wundprodukten werden die Fasern in eine flächige Matrix überführt. Durch die Aufnahme von Flüssigkeit aus dem Wundexsudat werden die Fasern nach und nach in ein Gelkissen umgewandelt, das die Flüssigkeit hält und nicht wieder freigibt. Dabei sind die Fasern so aufgebaut, dass das Wundexsudat nur in vertikaler Richtung aufgenommen wird. Dies bedeutet dass, solange die Kapazität reicht, das Exsudat nicht über den Wundrand fließt. Auf diese Weise kann eine Wundrandmazeration effektiv verhindert werden.

Besonders bevorzugt handelt es sich bei den hydroaktiven Polymeren um Superabsorbierende Polymere. Superabsorbierende Polymere (SAP) sind Kunststoffe, die in der Lage sind, ein Vielfaches ihres Eigengewichts - bis zum 1000-fachen - an Flüssigkeiten aufzusaugen. Chemisch handelt es sich dabei um ein Copolymer aus Acrylsäure (Propensäure, C₃H₄O₂) und Natriumacrylat (Natriumsalz der Acrylsäure, NaC₃H₃O₂), wobei das Verhältnis der beiden Monomere zueinander variieren kann. Zusätzlich wird ein so genannter Kernvernetzer (Core-Cross-Linker, CXL) der Monomerlösung zugesetzt, der die gebildeten langkettigen Polymermoleküle stellenweise untereinander durch chemische Brücken verbindet (sie "vernetzt"). Durch diese Brücken wird das Polymer wasserunlöslich. Beim Eindringen von Wasser oder wässrigen Salzlösungen in die Polymerpartikeln quellen diese auf und straffen auf molekularer Ebene dieses Netzwerk, so dass das Wasser ohne Hilfe nicht mehr entweichen kann.

Die superabsorbierenden Polymere können in dem erfindungsgemäßen Hygiene- oder Pflegeartikel in Form eines Granulats, eines Pulvers, einer Schüttung, eines Presslings, eines Schaums, in Form von Fasern, eines Fasergewirkes, -geleges oder -vlieses und/oder einer Faserwatte vorliegen.

Die Superabsorber-Teilchen können in Pulver- oder Granulatform mit einer Partikelgröße zwischen 100 und etwa 1000 µm vorliegen.

Wie die Anmelderin in früheren Patentanmeldungen gezeigt hat, sind superabsorbierende Polymere in der Lage, große Mengen an Exsudat aufzunehmen und zu binden. Sie reduzieren so den Anteil an pathologischem Exsudat in der Wunde und fördern damit die Wundheilung.

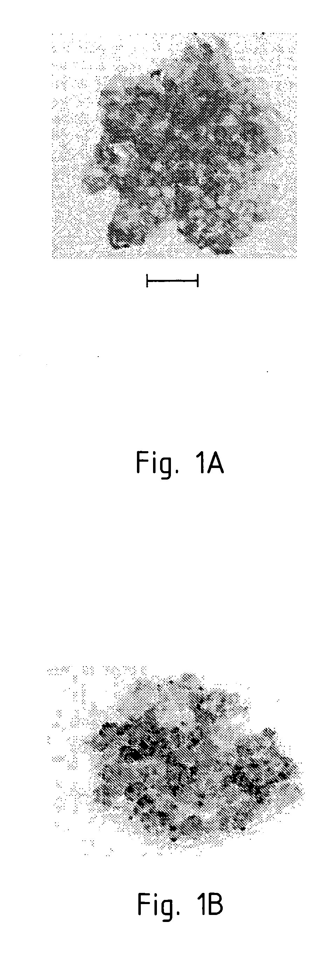

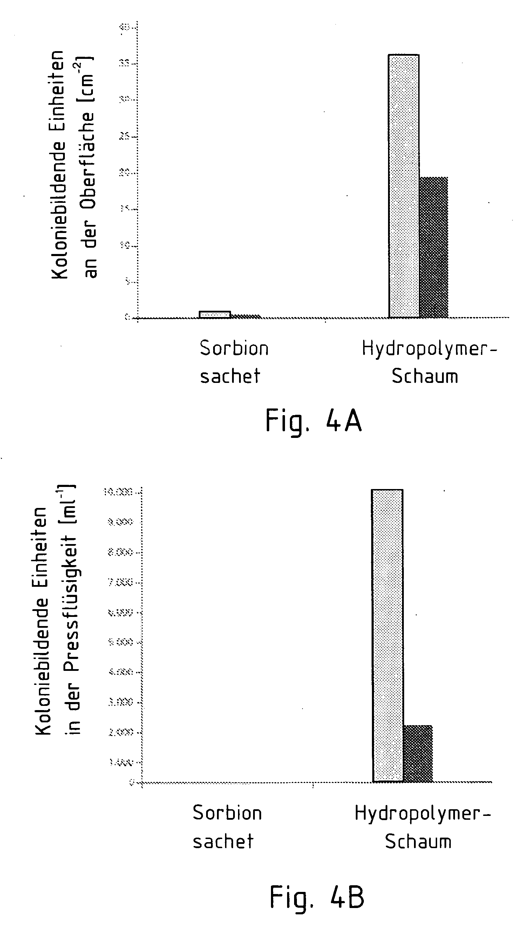

Die besagten hydroaktiven Polymere - insbesondere die Superabsorbierenden Polymere - binden jedoch nicht nur Flüssigkeiten, sondern auch Bakterien, Proteine und andere Biomoleküle, wie Untersuchungen der Anmelderin erstmals gezeigt haben (siehe Figs. 1 und 4).

Dieser Effekt ergänzt sich ideal mit dem bakteriziden Effekt der ebenfalls in dem erfindungsgemäßen Hygiene- oder Pflegeartikel vorgesehenen Bakteriophagenzubereitung.

Ein erfindungsgemäßer Hygiene- oder Pflegeartikel, aufweisend einen Anteil an superabsorbierenden Polymeren, und eine Zubereitung aufweisend Bakteriophagen oder mindestens einen Bestandteil derselben trägt also nicht nur dazu bei, die Anzahl an pathogenen Erregern in der Wunde durch Aufnahme der Bakterien mittels SAP und/oder durch Lysis der Bakterien durch die Bakteriophagenzubereitung aktiv zu reduzieren, sondern nimmt auch aktiv die entstandenen Lysate auf, und zwar insbesondere die darin enthaltenen Endotoxine.

Letzteres trägt auch zu einer Entlastung des Immunsystems bei, das ansonsten die entstehenden Endotoxine handhaben und entsorgen müsste.

Ein weiterer Vorteil eines erfindungsgemäßen Hygiene- oder Pflegeartikels, insbesondere Wundpflegeartikels, liegt darin, dass er nicht nur zur Behandlung von Wunden einsetzbar ist, die mit antibiotiokaresistenten Keimen infiziert sind, sondern dass er auch in solchen Fällen einsetzbar ist, in welchen eine Antibiotikatherapie aus anderen Gründen nicht angezeigt ist. Dies trifft z.B. zu
- für Schwangere, die in der Regel nur mit einem sehr eingeschränkten Antibiotikaspektrum (insbesondere Erythromycin) behandelt werden dürfen
- für Personen mit einer Antibiotikaallergie
- für Personen mit Leberschäden oder systemischen Defekten, bei denen bei einer Antibiotikatherapie eine metabolische Intoxikation zu befürchten ist

Ferner kann ein erfindungsgemäßer Hygiene- oder Pflegeartikel, insbesondere Wundpflegeartikel, so ausgestaltet sein, dass er nur bestimmte, pathogene Erreger bekämpft, nicht jedoch nicht pathogene Erreger. Dies kann über eine gezielte Auswahl der verwendeten Phagentypen bzw. ihrer Bestandteile erfolgen, da Bakteriophagen in der Regel nur ein sehr enges Wirtsspektrum aufweisen. Antibiotika weisen hingegen häufig eine Breitbandwirkung auf und unterschneiden daher nicht zwischen pathogenen und nicht pathogenen Keimen.

Letzteres ist insbesondere bedeutsam vor dem Hintergrund der Erkenntnis, dass - während eine nicht chronische Wunde in der Regel mit einer sogenannten "Normalflora" bakteriell kolonisiert ist, neuere Erkenntnisse dafür sprechen, dass eine chronische Wunde stets kritisch kolonisiert ist, d.h. bereits einen Anteil an pathogenen Keimen aufweist, auch wenn sie noch keine Anzeichen für eine Infektion oder eine Entzündung zeigt. Es gibt überdies Anzeichen dafür, dass besagte kritische Kolonisation einerseits eine Vorstufe für eine weitere Verschlechterung des Wundzustandes - d.h. eine chronische Entzündung - darstellt, und andererseits eine Verbesserung des Wundzustandes verhindert.

Die besagte kritische Kolonisierung wäre jedoch bereits eine Indikation für eine antipathogene Therapie, wenn letztere in der Lage wäre, zwischen pathogenen und nicht pathogenen Keimen zu differenzieren. Aufgrund des oben Gesagten kommt eine Antibiotikatherapie in diesem Falle nicht in Frage, wohl aber eine Bakteriophagentherapie.

Ein weiterer Vorteil eines erfindungsgemäßen Hygiene- oder Pflegeartikels, insbesondere Wundpflegeartikels, liegt darin, dass dieser länger auf der Wunde verbleiben kann als herkömmliche Wundauflagen. Grundsätzlich ist gerade dann, wenn der Kolonisierungsgrad einer Wunde nicht genau bekannt ist, ein häufiger Verbandwechsel erforderlich, um mim Fall einer Infektion schnell eingreifen zu können. Der erfindungsgemäße Wundpflegeartikel macht einen solchen häufigen Wechsel verzichtbar, da er die Kontrolle von Infektionen ermöglicht, und trägt so dem Bedürfnis nach Verlängerung der Wundruhephasen Rechnung, wie sie von Fachleuten im Rahmen der modernen Wundpflege insbesondere bei Diabetes-spezifischen chronischen Wunden gefordert wird.

Ein weiterer Vorteil des erfindungsgemäßen Hygiene- oder Pflegeartikels ist, dass er die Verwendung von Silber verzichtbar macht. In Hygiene- oder Pflegeartikeln aus dem Stand der Technik kommt häufig Silber bzw. Silberionen zum Einsatz, um ein bakterielles Wachstum zu unterbinden. Zwar gilt Silber als nicht toxisch, allerdings ist bislang ungeklärt, ob sich Silber im menschlichen Körper anreichert und ggf. zu Langzeitfolgen führt.

Ein weiterer Vorteil des erfindungsgemäßen Hygiene- oder Pflegeartikels ist, dass er sich insbesondere für die Behandlung von Verbrennungswunden eignet, die einerseits stark exsudieren (was den Einsatz von SAP sinnvoll macht), andererseits nicht oder kaum noch durchblutet werden, was eine systemische Antibiotikatherapie erschwert. Verbrennungswunden sind jedoch äußert anfällig für Infektionen, insbesondere für die im Krankenhausbereich häufig anzutreffenden multiresistenten Keime, so dass hier eine topische Phagentherapie eine neue, erfolgversprechende Option darstellt.

Ein weiterer Vorteil des erfindungsgemäßen Hygiene- oder Pflegeartikels liegt in einer synergistischen Reduktion der entzündungsbedingten Geruchsbildung, wie sie bei chronischen Wunden häufig anzutreffen ist. Durch die Aktivität der Bakteriophagen wird die für die Geruchsbildung verantwortliche Stoffwechselaktivität der Bakterien, die insbesondere Buttersäure freisetzen, gemindert. Gleichzeitig nehmen die superabsorbierenden Polymere sowohl bereits entstandene Geruchsstoffe als auch Exsudat und Wasser aus der Wunde auf und binden diese. Durch die Trockenlegung der Winde werden überdies die Wachstumsbedingungen für die geruchserregenden Bakterien verschlechtert, was zu einer weiteren Geruchsminderung führt.

Ein weiterer Vorteil des erfindungsgemäßen Hygiene- oder Pflegeartikels liegt darin, dass der Wundstoffwechsel durch die selektive Ausschaltung pathogener Keime normalisiert wird, insbesondere toxische Metaboliten besagter Keime reduziert werden, was wiederum zu einer verbesserten Wundheilung führt.

Besonders bevorzugt ist dabei vorgesehen, dass die Bakteriophagen ausgewählt sind aus der Gruppe enthaltend native Bakteriophagen, rekombinante Bakteriophagen, deaktivierte Bakteriophagen, und/oder modifizierte Phagen.

Besagte rekombinate Phagen können z.B. mit einem Gen für einen Fluoreszenzmarker ausgestatte sein, so z.B. für GFP. Die von den Phagen infizierten Bakterien exprimieren dannneben den Phagenproteinen - auch den besagten Fluoreszenzmarker, der bei der Lyse frei wird und mit Hilfe von UV-Licht nachgewiesen werden kann. Auf diese Weise kann der Erfolg der Phagentherapie kontrolliert werden; insbesondere kann gezeigt werden, ob die verwendeten Phagen spezifisch für die die Infektion auslösenden Bakterien sind; ist dies nicht der Fall, kann ein anderer Phagentyp verwendet werden.

Besagte modifizierte Phagen können z .B. an Magnetpartikel gekoppelt sein, um sie mit Hilfe eines Magneten nach der Behandlung aus der Wunde entfernen zu können, bzw. ihr Eindringen in die Wunde zu forcieren. Solche Magnetpartikel sind in der DE102007030931 der Anmelderin der vorliegenden Anmeldung beschrieben, auf deren Inhalt hier vollumfänglich Bezug genommen wird

Mit Hilfe eines erfindungsgemäßen Hygiene- oder Pflegeartikels kann die Ausbildung eines bakterienbedingten Biofilms, der z.B. aus bakteriellen Mucopolysacchariden bestehen kann, reduziert oder unterbrochen werden, was es ermöglicht, die Wunde gründlich zu reinigen bzw. der Wunde pflegende oder die Wundheilung fördernde Stoffe zuzuführen, so z.B. nutritive, desinfizierende bzw. dekontaminierenden und/oder Proteasen hemmend wirkende Wirkstoffe und/oder Wirkstoffkomplexe, wie sie z.B. in der DE102007030931 der Anmelderin der vorliegenden Erfindung beschrieben sind.

Bei Verwendung des erfindungsgemäßen Wundpflegeartikels ist auch eine frühzeitigere Applikation einer Vakuumtherapie möglich, wie sie z.B. in den Schriften DE202004017052, W02006048246 und DE202004018245 der Anmelderin der vorliegenden Erfindung beschrieben ist eine solche Therapie ist gerade bei stark exsudierenden Wunden angezeigt und führt bei diesen zu großen Erfolgen, verbietet sich jedoch, solange die Wunde infiziert ist.

Grundsätzlich tragen all diese Vorteile dazu bei, dass die Granulation der Wunde und eine anschließende Epithelisierung gefördert und so die Wundheilung beschleunigt wird. Weiterhin ist bevorzugt vorgesehen, dass der mindestens eine Bestandteil der Bakteriophagen ausgewählt sind aus der Gruppe enthaltend Lysine, Adhäsine, Spikeproteine, und/oder Grundplattenproteine. All die besagten Bestandteile können auch rekombinanter Herkunft, d.h. biotechnologisch hergestellt sein.

Bei besagten Lysinen (auch als Endolysine bezeichnet) handelt es sich um die phagenspezifischen lytischen Enzyme, die die Lyse des von einem Phagen befallenen Bakteriums induzieren.

Bevorzugte Beispiele für solche Lysine sind z.B. PlyG Lysin, gp37 aus dem Phagen T4, gp38 aus dem Phagen T2, Endolysin aus dem Bacillus amyloliquefaciens-Phagen, GBS- (Group B Streptococcal Phage)-Lysine, und/oder LysK aus Bakteriophage K.

Bei besagten Adhäsinen handelt es sich um Proteine, mit deren Hilfe die Phagen an das jeweilige Wirtsbakterium anbinden. Ähnliches gilt für die besagten Spikeproteine und Grundplattenproteine.

Ferner ist erfindungsgemäß vorgesehen, dass es sich bei den superabsorbierenden Polymeren um Polymere in Pulver- oder Granulatform handelt.

Superabsorbierende Polymere in dieser Darreichungsform weisen eine Vielzahl von Vorteilen auf uns sind z.B. aus der WO0152780 und der W003094813 der Anmelderin der vorliegenden Erfindung bekannt.

Alternativ ist vorgesehen, dass es sich bei den Superabsorbierenden Polymeren um Polymere in Faser-, Garn-, Watte- Vlies- oder Gewebeform handelt.

Die in dem vorliegenden vorgeschlagene Verwendung von Superabsorbierenden Polymeren in Faser- oder Garnform weist gegenüber partikulären superabsorbierenden Polymeren eine Reihe von Vorteilen auf:
i) So haben besagte Fasern oder Garne einen Dochteffekt. Auf diese Weise können sie bei Kontakt mit einer Flüssigkeit sehr viel schneller diese Flüssigkeit aufnehmen und binden als dies partikuläre superabsorbierende Polymere können.
ii) überdies können die Flüssigkeitssströme lenken bzw. richten. Auf diese Weise kann z.B. eine Wundrandmazeration verhindert werden.
iii) Sie können anders als partikuläre superabsorbierende Polymere zu Vliesen, Geweben oder ähnlichem verarbeitet werden. Auf diese Weise kann z.B. die Hülle einer Wundauflage aus diesen Fasern oder Garnen gefertigt sein, und die superabsorbierenden Eigenschaften können so sehr viel näher an den Wundgrund gebracht werden.
iv) Die Gefahr, dass superabsorbierende Materialien in der Wunde verbleiben, ist bei einem Gewebe aus superabsorbierenden Garnen sehr viel geringer als bei partikulären superabsorbierenden Polymeren. Auch dies trägt dazu bei, dass die superabsorbierenden Eigenschaften so sehr viel näher an den Wundgrund gebracht werden können
v) Da in vielen Fällen auf ein gesondertes Trägermaterial verzichtet werden kann, kann der Anteil an superabsorbierenden Materialien in einem Hygiene- oder Pflegeartikel, insbesondere einem Wundpflegeartikel erheblich erhöht werden; im Extremfall kann er sogar einen Gewichtsanteil von 100 % annehmen.
vi) Besagte Fasern oder Garne bzw. die daraus hergestellten Produkte besitzen eine sehr viel höhere Weichheit und eine geringere Abrasivität als die entsprechenden partikulären superabsorbierenden Polymere.
vii) Besagte Fasern oder Garne lassen sich zu einem Gefüge verarbeiten, ohne dass - wie es bei partikulären superabsorbierenden Polymeren erforderlich ist - ein Kleber oder ein Schweissverfahren verwendet werden muß. Dies hat sowohl in Bezug auf die Reinheit des Produkts als auch in Bezug auf die Pharmakologie und etwaige Allergenität erhebliche Vorteile.
viii) Im Gegensatz zu partikulären superabsorbierenden Polymere läßt sich die Dimensionierung besagter Fasern oder Garne sehr viel genauer steuern und kontrollieren, was einerseits zu Verhinderung von Stäuben führt, wie sie bei Verwendung von partikulären superabsorbierenden Polymere häufig entstehen., und was andererseits die Produktqualität (Homogenität und Reproduzierbarkeit) wesentlich erhöht.
ix) Aufgrund der fehlenden Ausbildung von Stäuben kann ggf. auch auf die Verwendung einer gesonderten Hülle verzichtet werden.
x) Besagte Garne oder Fasern können in aus dem Stand der Technik bekannten Airlaids, die partikuläre superabsorbierenden Polymere enthalten, die Trägerfasern des Airlaids ersetzen, um so den Anteil an superabsorbierenden Materialien in einem Hygiene- oder Pflegeartikel, insbesondere einem Wundpflegeartikel und damit die gesamte Absorptionskapazität - zu erhöhen.

Jegliche zwei- oder dreidimensionale Anordnung der Fasern oder Garne ist hier denkbar. So können die Fasern oder Garne gerichtet oder ungerichtet (Wirr-Warr), in mehreren Lagen oder sonstwie angeordnet sein.

Superabsorbierende Fasern aus Polyacrylaten werden z.B. von der Firma Technical Absorbents unter dem Handelsnamen "Oasis Super Absorbent Fibre" angeboten und vertrieben. Sie weisen wie alle Superabsorbierenden Polyacrylate eine sehr hohe Aufnahmekapazität für Flüssigkeiten auf. Diese Fasern können z.B. in Form eines Vlieses, eines Airlaids, eines Gewebes, eines Airlaids und/oder eines Nonwoven vorliegen. Solche Fasern sind z.B. aus der DE 69807337 bekannt

Die besagten Fasern aus superabsorbierendem Material sind jedoch in der Regel sehr brüchig, da das verwendete Polyacrylat-Material eine hohe Sprödheit aufweist. Aus diesem Grunde können die Fasern eine gewisse Länge nicht überschreiten und daher auch nicht ohne weiteres zu einem Garn verarbeitet werden.

Die WO0106047 beschreibt hingegen Garne aufweisend superabsorbierende Polymere. Diesen Garnen liegt ein besonderes Herstellungsverfahren zugrunde, bei welchem die oben genannten superabsorbierenden Fasern mit stützenden Fasern aus einem stärkeren Material gemischt und anschließend zu einem Garn versponnen werden. Bei besagten stützenden Fasern handelt es sich z.B. um Fasern aus Polyester, Polypropylen, Nylon, Baumwolle, Viskose oder ähnlichem Material. Das so hergestellte Garn kann dann zu einem Gewebe, Gelege, Gestrick und/oder Gewirke verarbeitet werden. Ebenso können die Fasern aber auch mit elastischen Fasern, beispielsweise aus Elasthan verarbeitet werden.

Bislang ist jedoch ausschließlich die Verwendung solcher Garne für Kabel, insbesondere Unterwasserkabel, beschrieben, so z.B. in der EP1072698, die vom selben Anmelder stammt wie die oben diskutierte WO0106047.

Bevorzugt ist dabei vorgesehen, dass die in Faserform vorliegenden superabsorbierenden Polymere mindestens teilweise in Form einer Watte, eines Vlieses, eines Airlaids und/oder eines Nonwoven vorliegen.

Hierzu werden bevorzugt Fasern mit mittleren Längen von 5 - 50 mm verwendet. Die Herstellung erfolgt mit bekannten Methoden, wie z.B. dem Kardieren oder dem Airlaid-Verfahren. Dabei können die Superabsorbierenden Fasern alleiniger Bestandteil des jeweiligen Materials sein. Bevorzugt ist jedoch vorgesehen, dass der Hygiene- oder Pflegeartikel überdies einen Anteil an stützenden Fasern aufweist, die auch in feuchtem Zustand die Integrität der Wundauflage gewährleisten, wobei besagte Fasern ausgewählt sind aus der Gruppe enthaltend
a) Cellulosefasern,
b) Viskosefasern,
c) Alginatfasern, und/oder
d) Polyester-, Polyolefine-, Polyurethan-, Polyvinylalkohol- oder Polymaidfasern, bzw. Mischungen oder Copolymere derselben

Auf diese Weise lassen sich die Absorptionseigenschaften des betreffenden Materials, aber auch andere Eigenschaften wie z.B. Dehnbarkeit, Reißfestigkeit, Verhalten bei Durchnässung und dergleichen, genau steuern.

Dabei kann insbesondere vorgesehen sein, dass ein Gewebe vorgesehen ist, bei dem z.B. die Kettfäden aus superabsorbierenden Garnen gefertigt sind, während die Schussfäden aus anderen Garnen bestehen, die z.B. stützende Fasern gemäß der obigen Aufzählung enthalten. Ein solches Gewebe weist gerichtete hydroaktive Eigenschaften auf, d.h. es nimmt Flüssigkeit in einer Richtung auf und leitet sie weiter.

Bevorzugt können die Fasern mindestens teilweise in Form eines Vlieses, eines Gewebes, eines Gewirkes, eines Airlaids und/oder eines Nonwoven vorliegen.

Bei den verwendeten Bakteriophagen - bzw. den Quellen für die Bestandteile der Bakteriophagen kann es sich grundsätzlich um Mitglieder folgender biologischer Taxa handeln:

**Tabelle 1**

| **Gruppe** | **Ordnung** | **Familien** | **Gattung/Arten** |
|---|---|---|---|
| dsDNA-Bakteriophagen | *Caudovirales* | *Myoviridae* | T4-ähnliche Viren |
| | | | P1-ähnliche Viren |
| | | | P2-ähnliche Viren |
| | | | Mu-ähnliche Viren |
| | | | SPO1-ähnliche Viren |
| | | | ϕH-ähnliche Viren |
| | | *Siphoviridae* | λ-ähnliche Viren |
| | | | T1-ähnliche Viren |
| | | | T5-ähnliche Viren |
| | | | L5-ähnliche Viren |
| | | | c2-ähnliche Viren |
| | | | ψM1-ähnliche Viren |
| | | | ϕC31-ähnliche Viren |
| | | | N15-ähnliche Viren |
| | | *Podoviridae* | T7-ähnliche Viren |
| | | | P22-ähnliche Viren |
| | | | Φ29-ähnliche Viren |
| | | | N4-ähnliche Viren |
| | | *Tectiviridae* | |
| | | *Corticoviridae* | |
| | | *Plasmaviridae* | |
| | | *Podoviridae* | |
| | | *Lipothrixviridae* | |
| | | *Rudiviridae* | |
| | | *Fuselloviridae* | *Salterprovirus* |
| ssDNA-Bakteriophagen | | *Inoviridae* | |
| | | *Microviridae* | |
| dsRNA-Bakteriophagen | | *Cystoviridae* | |
| ssRNA-Bakteriophagen | | *Leviviridae* | |

In einer besonders bevorzugten Ausgestaltung der Erfindung ist vorgesehen, dass die verwendeten Bakteriophagen - bzw die Quellen für die Bestandteile der Bakteriophagen - ausgewählt sind aus der Gruppe enthaltend Myoviridae und/oder T-Phagen.

Die Familie Myoviridae umfasst sechs Gattungen von Bakteriophagen mit einem Molekül doppelsträngiger DNA als Genom. Die Mitglieder dieser Familie besitzen ein ikosaedrisches Kapsid und ein kontraktiles Schwanzteil. Innerhalb der Ordnung Caudovirales zeichnen sich die Myoviridae durch besonders große, teilweise langgestreckte Kapside (z.B. T4-Phage: 111x78 nm) und ein sehr großes Genom (34-169 kBp) aus. Der wichtigste Vertreter ist der schon früh entdeckte T4-Phage (Enterobacteria phage T4), dessen besondere Morphologie und genetische Eigenschaften in der molekularbiologischen Forschung eine herausragende Rolle spielte. Bevorzugt sind hier die geradzahligen T-Phagen, insbesondere die T4-ähnlichen Phagen.

Es wurde festgestellt, dass Mitglieder dieser Familie viele gramnegative Bakterien, insbesondere die meisten Enterobakterien und deren verwandte Verwandte, infizieren. Die meisten geradzahligen T-Phagen verwenden 5-Hydroxymethylcytosin anstelle vom Cytosin in ihrer DNA, um sich gegen die bakteriellen Restriktionsenzyme zu schützen, wodurch sie überdies ihr Wirtsspektrum erweitern.

Die geradzahligen T-Phagen teilen eine einzigartige Fähigkeit, die erheblich zu ihrem weitverbreiteten Auftreten in der Natur und zu ihrem evolutionären Erfolg beiträgt. Sie sind in der Lage, den Zeitpunkt der Lysis abhängig von der Abundanz ihrer bakteriellen Wirte im Milieu zu steuern.

Wenn z.B. E. coli Zellen einzeln mit T4 angesteckt werden, lösen sie bei Körpertemperatur nach 25-30 Minuten auf und geben pro Zelle ungefähr 100-200 neue Phagen frei. Wenn zusätzliche geradzahligen T-Phagen die Zelle mehr als 4 Minuten nach der Ausgangsinfektion angreifen, löst sich die Zelle nach der oben angebebenen Zeit auf. Stattdessen fährt sie fort, für einen Zeitraum von 6 Stunden weitere Phagen zu bilden, wobei der genaue Zeitpunkt der endgültigen Lysis durch die Mange der superinfizierenden Phagen gesteuert wird. Diese Verzögerung wird als "Lysishemmung" bezeichnet.

Aus all diesen Gründen sind Myoviridae und/oder T-Phagen, bevorzugt geradzahlige T-Phagen, besonders bevorzugt T4-Phagen, ausgezeichnete Kandidaten im Rahmen der erfindungsgemäßen Verwendung.

In der folgenden Tabelle sind besonders bevorzugt in Frage kommende Bakteriophagen aufgelistet:

**Tabelle 2**

| **Art** | **Wirt** |
|---|---|
| T4-Phage | *Enterobakterien* |
| (12 Subtypen, darunter auch T2-Phage) | |
| Enterobacteria Phage P1 | *Enterobakterien* |
| Aeromonas Phage 43 | *Aeromonas* |
| Enterobacteria Phage P2 | *Enterobacter* |
| Haemophilus Phage HP1 | *Haemophilus* |
| Enterobacteria Phage Mu | *Enterobacter* |
| Bacillus Phage SPO1 | *Bacillus subtilis* |
| Halobacterium Phage ϕH | *Halobacterium* |
| Halobacterium Phage Hs1 | |
| Bakteriophage K | *Staphylococcus* |
| MSA | *Staphylococcus aureus* |
| LP65 | *Lactobacillus plantarum* |
| A511 | *Listeria* |
| Bacillus amyloliquefaciens-Phage | *Bacillus amyloliquefaciens* |
| GBS Phagen (Group B streptococcus Phagen): | *Streptococcus* |
| | |
| NCTC 11261 | |
| NCTC 11237b | |
| Bacteriophage B30 | |
| Phage 3396 | |
| | |
| ϕ11, ϕ12, ϕ20, ϕ812, ϕ131 | *Staphylococcus aureus* |
| SK311 | *Staphylococcus aureus* |
| U16 | *Staphylococcus aureus* |
| Twort | *Staphylococcus aureus* |

Weitere, im Rahmen der vorliegenden Erfindung in Frage kommende Bakteriophagen sind die Phagen Barnyard, Bethlehem, Bxb1, Bxz1, Bxz2, Che8, Che9c, Che9d, CJW I, Cooper, Corndog, D29, Halo, ES18, phiK02, G, HK022, HK97, L5, LLIJ, MP9, MP40, Mu, N15, N4, NPV1, Omega, P22, PAU, PBI1, PG1.htm, Rosebush, SP6, SPO1, TM4, U2, Wildcat, Bastile, MP15, MP45, MP9, PBS, 186, 2389, 7201, 933W, A118, APSE-1, c2, Cp-1, D3, phi-C31, HK620, Lambda, Mx8, P1 (*Mycoplasma*), P2, P27, P4, PM2, Sfi19, Sfi21, T3, T4, T7, P58, K5, K1, PM2, P22, K1-5, ENB6, IRA, SP6, T12, RZh, und H4489a bzw. Bestandteile derselben.

Weitere im Rahmen der vorliegenden Erfindung in Frage kommende Bakteriophagen (bzw. Bestandteile derselben) sind solche Phagen, die Bakterien der folgenden Taxa infizieren und/oder lysieren können: Escherichia, Shigella, Salmonella, Erwinia, Yersinia, Bacillus, Vibrio, Legionella, Pseudomonas, Neisseria, Bordetella, Helicobacter, Listeria, Agrobacterium, Staphylococcus, Streptococcus, Enterococcus, Clostridium, Corynebacterium, Mycobacterium, Treponema, Borrelia, Francisella und Brucella. Der Fachmann wird durch diese Aufzählung in die Lage versetzt, gegen die hier aufgezählten Bakterien gerichtete und durch den schutz- und Offenbarungsgehalt dieser Anmeldung erfasste Phagen aus der Fachliteratur zu entnehmen, ohne hierfür erfinderisch tätig zu sein.

Bei den erwähnten rekombinanten Bakteriophagen handelt es sich beispielsweise um A511::luxAB (Loessner et al., Appl. Environ. Microbiol., 62(4):1133-40, 1996), phAE40 (Riska et al., J. Clin. Microbiol., 35(12):3225-31, 1997), PhiV10::luxABcamA1-23 (Waddell, und Poppe, FEMS Microbiol. Lett. 182:285-89, 2000), K1-5::luxAB (Loessner et al., Appl. Environ. Microbiol., 62(4): 1133-40, 1996), M13, der entweder BglII oder λ S Holin-gene codiert (Hagens und Blasi, Letters in Applied Microbiology 37:318-323, 2003) bzw. um Bestandteile derselben.

Weiterhin ist bevorzugt vorgesehen, dass der Wundpflegeartikel einen Wundexsudate absorbierenden Körper sowie eine mindestens abschnittsweise um diesen Körper angeordnete Hülle aufweist.

Dabei weist der Wundexsudate absorbierende Körper mindestens ein Material auf, das ausgewählt ist aus der Gruppe enthaltend eine Matte, insbesondere aus einem Airlaid aus besagten Garnen oder Fasern aus superabsorbierenden Polymeren mit eingearbeiteten superabsorbierenden Polymeren, und/oder eine lose Füllung aus superabsorbierenden Polymeren. Besagte Airlaidmatte kann bevorzugt einen im wesentlichen flachen Materialabschnitt aus Absorptionsmaterial aufweisen, der z. B. aus einem aufsaugenden Vlies aus den genannten Fasern mit darin verteilten superabsorbierenden Polymeren besteht.

Dieser Wundexsudate absorbierende Körper kann der absorbierenden Einlage entsprechen, die in einer Wundauflage der Anmelderin der vorliegenden Erfindung enthalten ist, wie sie beispielsweise in der W003094813, der WO2007051599 und der WO0152780 offenbart ist und unter dem Handelsnamen "sorbion sachet" vertrieben wird. Der Offenbarungsgehalt der genannten Schriften sei dem Offenbarungsgehalt dieser Schrift vollumfänglich beigefügt.

Der Wundexsudate absorbierende Körper kann in einer anderen Ausgestaltung ebenso einen Kern bilden, der - ggf. flockenartige - Fasern oder Garne aus superabsorbierenden Polymeren sowie superabsorbierenden Polymeren in Granulatform aufweist, wobei die Granulate an die Fasern bzw. Garne in mehreren Höhen angeklebt bzw. angeschweißt sind, und die Granulate über mehr als 50 % der gesamten Bauhöhe wenigstens eines Abschnitts des Kerns verteilt sind, wobei vermengte Bereiche von Granulat und Fasern vorliegen. Der Gewichtsanteil der superabsorbierenden Polymeren kann dabei bevorzugt im Bereich zwischen 10-25 Gew.-% liegen. Ähnliche Konstruktionen sind aus herkömmlichen Inkontinenzmaterialien bekannt und wie Hygienebinden für ihre polsternden Eigenschaften bekannt.

Um besagten Kern herum kann eine Hülle angeordnet sein, die in Bereichen überlappend angeordnet ist, und der z.B. eine Klebenaht überdeckt bzw. Teil derselben ist.

Ebenso kann innerhalb der Hülle ein Abschnitt eines hydrophoben und/oder wasserabweisenden bzw. wasserundurchlässigen Materials vorgesehen sein, der Durchnässungs- oder Wäscheschutz fungiert.

Der Wundexsudate absorbierende Körper kann in einer anderen Ausgestaltung ebenso mindestens eine flache Lagen aufweisend Fasern oder Garne aus superabsorbierenden Polymeren enthalten, an welche superabsorbierende Polymere in Granulatform geklebt sind. Dadurch ergibt sich in einer bevorzugten Ausgestaltung ein Aufbau des Körpers, der wenigstens drei Schichten aufweist, wobei zwei Deckschichten eine Schicht aufweisend superabsorbierende Polymere umgeben.

Dabei liegen in der Ebene keine Vermengungen von Fasern und superabsorbierenden Polymeren vor; sondern lediglich fixierte Benachbarungen beider Materialien. Die ggf. vorgesehenen mehreren lagen können dabei in einer bevorzugten Ausgestaltung auch durch walzen, Pressen, Kalandrieren oder ähnliche Verfahren physisch miteinander verdichtet sein. Überdies kann der Körper sich wiederholende Musterungen oder Maserungen aufweisen, wie z.B. ein Karomuster, ein Stanzmuster oder dergleichen.

Der besagte Wundexsudate absorbierende Körper kann überdies ggf. eine Hülle aus einem durchlässigen Material aufweisen; diese kann in ihren Randbereichen unterschiedliche Verbindungen bzw. Nähte aufweisen, die insbesondere durch Klebungen erzeugt sind. So kann z.B. vorgesehen sein, dass der Verbindungsbereich zwischen dein beiden Seiten der Hülle an mindestens einer Seite des Wundexsudate absorbierenden Körpers - bevorzugt in Längsrichtung - schmaler ausgestaltet ist als an mindestens einer anderen Seite, so dass sich im ersteren Falle, anders als im letzteren Falle ein sich auffaltbarer Überstand ergibt.

Besagte Hülle kann bevorzugt aus einem Nonwoven aus Polypropylen mit einem Flächengewicht von 10-40 g/m² bestehen.

Bevorzugt kann überdies vorgesehen sein, dass der so beschriebene Wundexsudate absorbierende Körper an wenigstens einer Innerseite der ihn umgebenden Hülle fixiert ist, bevorzugt durch Klebungen.

Besonders bevorzugt ist vorgesehen, dass die in Garnform vorliegenden superabsorbierenden Polymere mindestens teilweise in Form eines Gewebes, eines Geleges, eines Gestricks und/oder eines Gewirkes vorliegen. Ebenso können die einzelnen Garne auch in Form eines Gitters miteinander verschweißt sein.

In dieser Form können die superabsorbierenden Garne z.B. als Hülle einer Wundauflage fungieren. Ebenso kann aus besagtem Material ein Verband ähnlich einer Mullbinde hergestellt werden. Es kann jedoch auch ein dreidimensionales Gewebe oder Gestrick hergestellt werden, dass als zentraler Absorptionskörper einer Wundauflage fungiert. Dabei können die Superabsorbierenden Garne alleiniger Bestandteil des jeweiligen Materials sein, sie können jedoch auch in Kombination mit anderen Fasern verwendet werden, so z.B. mit Zellstoff-, Viskose-, Baumwoll- und/oder Polyolefinfasern.

Auf diese Weise lassen sich die Absorptionseigenschaften des betreffenden Materials, aber auch andere Eigenschaften wie z.B. Dehnbarkeit, Reißfestigkeit, Verhalten bei Durchnässung und dergleichen, genau steuern.

Ferner ist erfindungsgemäß ein Wundpflegeartikel vorgesehen, der dadurch gekennzeichnet ist, dass die in Faser- und/oder Garnform vorliegenden superabsorbierenden Polymere Bestandteil einer flächigen Matte und/oder einer flächigen Wundauflage sind.

Ebenso ist erfindungsgemäß ein Hygiene- oder Pflegeartikel vorgesehen, der dadurch gekennzeichnet ist, dass die in Faser- und/oder Gasform vorliegenden superabsorbierenden Polymere Bestandteil eines dreidimensionalen Absorptionskörpers sind.

Ebenso ist erfindungsgemäß ein Wundpflegeartikel vorgesehen, der dadurch gekennzeichnet ist, dass die in Faser- und/oder Garnform vorliegenden superabsorbierenden Polymere Bestandteil einer Hülle einer Wundauflage sind.

Alternativ ist vorgesehen, dass die in Faser- und/oder Garnform vorliegenden superabsorbierenden Polymere Bestandteil eines Schaums sind. Bei diesem Schaum kann es sich z.B. um ein Material ausgewählt aus der Gruppe enthaltend thermoplastische Weichschäume, wie Polyurethan-, Polyamid- oder Polyetherschaum, Silikonschaum sowie Cellulose-Schaum oder Naturschwamm handelt.

Naturschwämme, beispielsweise der Klasse der Hornkieselschwämme (Demospongiae), weisen ähnlich wie technische Schaumstoffe ein Absorptionsvermögen für Flüssigkeiten auf. Überdies weisen sie wachstumshemmende Eigenschaften gegenüber Mikroorganismen auf, um sich vor dem Ansiedeln von sessilen Organismen zu schützen. Diese Eigenschaften können auch in Zusammenhang mit der Wundversorgung sinnvoll sein, um Bakterienwachstum in der Wundauflage und/oder in der Wunde zu verhindern. Ebenso weisen diese Schwämme wachstumshemmende Eigenschaften gegenüber Pilzen und Einzellern auf. Hinzu kommt, dass solche Schwämme Flüssigkeiten zu absorbieren imstande sind und sich daher für die Aufnahme von Exsudaten hervorragend eignen.

Besagter Naturschwamm kann in dünnen Scheiben, die z. B. durch thermisches Schneiden erzeugt worden sind, auf die Wunde aufgelegt sein.

Der Weichschaumstoff kann ggf. mehrlagig ausgestaltet sein, wobei die einzelnen Lagen bevorzugt Dicken zwischen 0,5 mm und 10 mm aufweisen können.

Der Weichschaumstoff kann offenzellig und geschlossenzellig ausgestaltet sein. Insbesondere in letzterem Falle kann vorgesehen sein, dass das Material Stanzungen und/oder Perforationen aufweist, welche den Flüssigkeitsein- und -durchtritt in bzw. durch den Weichschaumstoff beschleunigen. Überdies kann der Weichschaumstoff auch als Integralschaum ausgestaltet sein.

Dabei kann vorgesehen sein, dass die Superabsorbierenden Polymere in das Schaumstoffmaterial eingebracht sind. In besagtem Fall können die superabsorbierenden Fasern oder Garne bereits vor dem Aufschäumen des Schaumstoffs in das Ausgangsmaterial eingebracht werden. Alternativ können sie auch mittels Druck in das fertige Schaumstoffmaterial eingepresst werden.

Auf diese Weise wird die Absorptionskapazität des erfindungsgemäßen Hygiene- oder Pflegeartikels weiter erhöht. Überdies können die Fasern oder Garne eine Dochtwirkung aufweisen. Dies ist insofern vorteilhaft, da so gewährleistet ist, dass die Oberfläche des Hygiene- oder Pflegeartikels trocken bleibt und aufgenommenes Exsudat schnell nach innen weitergeleitet wird.

In ähnlicher Form können z.B. gefriergetrocknete Phagenzubereitungen in einen Weichschaumstoff eingebracht werden.

Weiterhin kann vorgesehen sein, dass die in Faser- und/oder Gasform vorliegenden superabsorbierenden Polymere auf eine Trägerlage aufgebracht sind.

Hier kann vorgesehen sein, dass die Fasern in einer Wirrwarr-Anordung ggf. beidseitig auf eine Lage aufgestreut werden, die zuvor mit einem adhäsiven Material beschichtet wurde. Hierbei kann es sich z.B. um Paraffin oder Glycerin handeln. Ebenso kann ein Gewebe aufweisend superabsorbierendes Garn auf besagte Lage aufgebracht sein. Mehrere dieser Lagen können z.B. in einem erfindungsgemäßen Hygiene- oder Pflegeartikel übereinander gelegt sein.

In ähnlicher Form können auch gefriergetrocknete Phagenzubereitungen auf eine Trägerlage aufgebracht werden.

Ebenso kann vorgesehen sein, dass der Hygiene- oder Pflegeartikel, insbesondere Wundpflegeartikel in Form eines Tupfers, eines Wundtuchs, einer Wundauflage, einer Wundkompresse, eines Wundpolsters, einer ggf. elastischen Wickel, einer Bandage, eines Pflasters oder eines Strumpfes vorliegt.

Ferner ist die Verwendung eines erfindungsgemäßen Wundpflegeartikels zur Behandlung von chronischen Wunden, akut blutenden Wunden, Verbrennungswunden und traumatisch erzeugten Wunden vorgesehen.

Ebenso ist die Verwendung eines erfindungsgemäßen Wundpflegeartikels zur operativen bzw. postoperativen Versorgung bzw. für militärische Zwecke vorgesehen.

Weiterhin ist ein Kit für die Akut-, Notfall- oder Militärmedizinische bzw. die chronische Versorgung vorgesehen, aufweisend einen erfindungsgemäßen Wundpflegeartikel.

### Varianten

Vorzugsweise besteht eine ggf. vorgesehene Hülle aus zumindest teilweise auch aus einem hydrophobem Material, beispielsweise aus Polypropylen oder aus einem hydrophob ausgerüsteten Naturmaterial, wie Baumwolle. Die hydrophoben Eigenschaften der Hülle verhindern das Verkleben mit der Wundoberfläche und tragen dazu bei, dass das Wundexsudat schneller ins Innere der Hülle gelangen kann.

Die Hülle kann auch aus einem anderen Kunststoff, insbesondere einer Polyurethan- oder Polyethylenfolie oder aus künstlicher Spinnenseiden Folie hergestellt sein.

Das Material der Hülle kann derart strukturiert sein, dass die Hülle eine raue Innenfläche und eine glatte Außenfläche aufweist. Vorzugsweise ist die raue Innenfläche der Hülle durch trichterförmige Perforationen gebildet, die sich jeweils in Richtung Innenfläche verjüngen und in eine freie Öffnungskante ("Auskragung") auslaufen. Diese raue Innenfläche wirkt den Verschiebungen des Inhaltes der Hülle entgegen, so dass auf eine Fixierung mit Klebepunkten verzichtet werden kann. Dementsprechend kann die glatte Außenfläche des Hüllenmaterials durch gewölbte, sich zwischen den Perforationen erstreckende Materialabschnitte gebildet sein. Ein solches Hüllenmaterial kann im Gegensatz zu einem beidseitig ebenen als "dreidimensional" bezeichnet werden, und ist aus z.B. aus der DE 102006017194 der Anmelderin der vorliegenden Anmeldung bekannt, auf deren Offenbarungsgehalt hier vollumfänglich verwiesen wird.

Bevorzugt ist überdies vorgesehen, dass die Hülle eines erfindungsgemäßen Hygiene- oder Pflegeartikels zumindest abschnittsweise eine adhäsive Beschichtung aufweist, und zwar bevorzugt auf der wundabgewandten Seite, mit deren Hilfe sie - z.B. mit einem Verband - im Wundbereich fixiert werden kann.

Weiterhin kann auch vorgesehen sein, dass die Hülle einen über die eigentliche Wunde überstehenden Bereich aufweist, der mit Klebestreifen zur Fixierung versehen ist.

Überdies kann auch auf der Wundabgewandten Seite des Wundpflegeartikels ein flächiger Materialabschnitt vorgesehen sein, der über die eigentliche Hülle hinausgeht und wenigstens in seinen Randbereichen eine der Haut zugewandte adhäsive Beschichtung z.B. in Form von Klebestreifen oder -flächen aufweist (sog. "Island Dressing").

Besagter flächiger Materialabschnitt kann insbesondere semiokklusive Eigenschaften aufweisen, d.h. er kann z.B. durchlässig für Feuchtigkeit sein, nicht aber für Bakterien.

Als Klebematerialien für die oben genannten Zwecke kommen bevorzugt physiologisch akzeptable adhäsive Agenzien in Frage, wie z.B. Hydrokolloidkleber oder medizinisch unbedenkliche Klebstoffe, wie lösungsmittelfreie, biokompatible Silikonkleber oder Polyacrylatkleber sein, die eine gute Beständigkeit gegen alle gängigen Sterilisationsverfahren aufweisen.

Der Polyurethanschaum lässt sich im Gegensatz zu anderen Kunststoffen, wie Polypropylen, Polytetrafluorethylen (Teflon) und Silikon gut kleben.

In einer besonderen Ausführungsform ist vorgesehen, dass der absorbierende Körper innerhalb der Hülle unsymmetrisch, d.h. überwiegend auf einer Seite angeordnet ist.

Ein solcher Wundpflegartikel lässt sich bevorzugt in Wundtaschen einsetzen, in denen beengte Raumverhältnisse herrschen. Dabei verbleibt der Abschnitt des Wundpflegeartikels, in dem sich der absorbierende Körper befindet, außerhalb der Wundtasche gezeigt. Durch die Kapillarkräfte werden Exsudate, die sich in einer solchen Wundtasche befinden, effektiv aufgenommen und auch hier die Wundheilung gefördert.

Bevorzugt ist weiterhin vorgesehen, dass der Wundpflegeartikel einen lateralen Einschnitt oder eine keilförmige Aussparung aufweist, dergestalt, dass sich die Ränder des Einschnitts bzw. der Aussparung überlappend anordnen lassen.

Bevorzugt ist weiterhin vorgesehen, dass der Wundpflegeartikel einen die Wunde ausfüllenden Abschnitt aufweist. Dieser kann z.B. so ausgestaltet sein, dass er bei Kontakt mit Exsudat aufquillt und die Wunde bis zum Wundboden ausfüllt.

Auf diese Weise ist der Wundpflegeartikel dreidimensional formbar, so dass sich eine konkave Form ergibt, um ihn z.B. für die Auflage an ein Gelenk, eine Extremität oder ein gekrümmtes Körperteil anzupassen. Die überlappend angeordneten Ränder des Einschnitts bzw. der Aussparung können dabei z.B. durch Klettverschlüsse, Druckköpfe, Klebestreifen oder andere geeignete Fixierungsmittel fixiert werden.

In einer anderen bevorzugten Ausgestaltung ist vorgesehen, dass der Wundpflegeartikel dreidimensional geformt ist, dergestalt, dass er für die Auflage an ein Gelenk, eine Extremität oder ein gekrümmtes Körperteil angepasst ist.

So kann der Wundpflegeartikel z.B. eine konkave Form aufweisen, um ihn z.B. für die Auflage an die Ferse eines Fußes oder an einen Ellenbogen eines Patienten anzupassen. Der absorbierende Körper, der im Inneren des Wundpflegeartikels angeordnet ist, kann dabei herausnehmbar gestaltet sein. Besonders von Vorteil ist diese Ausgestaltung, wenn der absorbierende Körper zuvor angefeuchtet wurde bzw. durch austretendes Exsudat angefeuchtet wird, da sich hier dann aufgrund der vorliegenden superabsorbierenden Polymere ein Gel ausbildet, das kühlend und polsternd wirkt und somit die schmerzfreie Lagerung des besagten Körperteils ermöglicht. Überdies passt sich der Wundpflegeartikel durch die Anfeuchtung noch besser den anatomischen Gegebenheiten an.

Bevorzugt ist überdies vorgesehen, dass der Wundpflegeartikel zumindest teilweise in einer gerollten Form vorliegt. Hier kann vorgesehen sein, dass der ursprünglich flächige Wundpflegeartikel gerollt und ggf. in seiner gerollten Form z.B. durch Nähen, Kleben oder Schweissen fixiert wird. Ein solcher Wundpflegeartikel eignet sich insbesondere für die Verwendung als Tamponade in Wundtaschen; er weist insbesondere eine Dochtfunktion für die aufzunehmenden Exsudate auf.

Bevorzugt ist in diesem Zusammenhang außerdem vorgesehen, dass der Wundpflegeartikel außerdem mindestens einen nutritiven, mindestens einen desinfizierenden bzw. dekontaminierenden und/oder mindestens einen Proteasen hemmend wirkenden Wirkstoff und/oder Wirkstoffkomplex aufweist.

Bei dem desinfizierend wirkenden Wirkstoff und/oder Wirkstoffkomplex kann es sich z.B. um eine Zusammensetzung aus mindestens einem Vitamin oder Vitaminderivat, einem Metallion sowie einem Detergenz handeln. Ebenso kann es sich dabei um ein BLIS (bacteriocin like inhibitory substance), um ein antimikrobielles Peptid, ein Antibiotikum, ein Silberpräparat oder um beschichtete magnetische Partikel handeln.

Die Kombination aus erfindungsgemäß verwendeten Bakteriophagen und desinfizierend wirkendem Wirkstoff und/oder Wirkstoffkomplex, insbesondere Antibiotika und/oder Silberpräparat ist besonders erfolgversprechend für die Behandlung von chronischen, ggf. infizierten Wunden.

Bei dem nutritiv wirkenden Wirkstoff und/oder Wirkstoffkomplex kann es sich um eine Zusammensetzung enthaltend mindestens die Bestandteile eines enteralen und/oder parenteralen Diätetikums handeln. Ebenso kann es sich dabei um mindestens ein Wirkelement ausgewählt aus der Gruppe enthaltend Insulin, rekombinantes Insulin, Proinsulin, einen insulinähnlichen Wachstumsfaktor (Insulin-like growth factor, IGF), ein Insulinmimetikum und/oder einen diabetikerspezifischen, nicht glucose- bzw. saccharosebasierenden Energieträger handeln.

Genauso kann es sich bei dem nutritiv wirkenden Wirkstoff und/oder Wirkstoffkomplex um ein Glycolyse-Enzym handeln, so z.B. um eine Hexokinase, die Glucose in Glucose-6-Phosphat überführt und so durch Einleitung der Glycolyse den bei Diabetikern häufig erhöhten Glucosespiegel reduziert.

Bei dem Proteasen hemmend wirkenden Wirkstoff und/oder Wirkstoffkomplex kann es sich um mindestens ein Wirkelement ausgewählt aus der Gruppe enthaltend Proteasehemmer, superabsorbierende Polymere, Antikörper gegen Matrix -Metallo-Proteasen (MMP, insbesondere gegen MMP 2, 7 und 9), Chelatoren für zweiwertige Kationen (insbesondere für Ca²⁺), Kollagen, beschichtete magnetische Partikel, Säuren, Puffer, nicht pathogene säureproduzierende Mikroorganismen, Probiotika und/oder Symbiotika handeln. Insbesondere kann dabei vorgesehen sein, dass in der Wundauflage eine Trägersubstanz vorgesehen ist, die zweiwertige Kationen (insbesondere Ca²⁺) bindet.

Weitere Zusammenhänge und Hintergründe zu den nutritiven, einen desinfizierenden bzw. dekontaminierenden und/oder Proteasen hemmend wirkenden Wirkstoffen und/oder Wirkstoffkomplexen sind in der DE102007030931 der Anmelderin der vorliegenden Anmeldung beschrieben, auf deren Inhalt hier vollumfänglich Bezug genommen wird. In der DE102007030931 sind auch weitere nutritive, desinfizierende bzw. dekontaminierende und/oder Proteasen hemmend wirkende Wirkstoffen und/oder Wirkstoffkomplexe beschrieben, die ebenfalls als in dieser Anmeldung offenbart gelten sollen.

Es kann überdies vorgesehen sein, dass die Wundauflage nekrolytische und/oder fibrinolytische Enzyme enthält. Ebenso kann sie Angiogenese- oder Epidermogenese-fördernde Wachstumsfaktoren enthalten (insbesondere aus der Gruppe der VEGF und EGF). Ebenso kann die Wundauflage Lockstoffe für Makrophagen enthalten, die die bei der Phagentherapie freiwerdenden Phagen und Bakterrienreste (insbesondere Endotoxine) phagocytieren.

Weiterhin ist bevorzugt vorgesehen, dass mindestens ein Abschnitt der Hüllenwand des Wundpflegeartikels ein Reservoir für besagte Phagenzubereitung und/oder mindestens einen nutritiven, einen desinfizierenden bzw. dekontaminierenden, einen Proteasen hemmend wirkenden, einen blutstillend wirkenden und/oder einen wundheilungsfördernden Wirkstoff und/oder Wirkstoffkomplex aufweist.

Besagtes Reservoir kann z.B. aus einer in die Hüllenwand eingearbeiteten Tasche bestehen. Ebenso kann das Reservoir aus einem mit besagtem Wirkstoff und/oder Wirkstoffkomplex getränkten Abschnitt der Hüllenwand bestehen, oder der Wirkstoff ist in besagten Abschnitt eingepresst.

Dem Wundpflegeartikel können Substanzen zugefügt sein, die den osmotischen Druck erhöhen können. Zu den Substanzen zählen z. B. Osmodiuretika, wie Mannitol.

Mineralische Ionenaustauscher, wie Zeolithe, Bentonite oder Montmarylinite, können ebenfalls Bestandteil des Wundpflegeartikels, insbesondere seiner Matte sein. Zeolithe können u. a. Schadstoffe, wie Schwermetalle, absorbieren. Überdies entfalten sie eine blutstillende Wirkung.

Ebenso kann der Artikel auch Aktivkohle enthalten.

Weiterhin kann der erfindungsgemäße Wundpflegeartikel auch in ein Wundversorgungssystem zur Wunddrainage unter Einsatz von Unterdruck eingebracht sein. Solche Systeme sind z.B. in den Schriften DE202004017052, WO2006048246 und DE202004018245 der Anmelderin der vorliegenden Erfindung offenbart, deren Offenbarungsgehalt der vorliegenden Erfindung als zugehörig betrachtet sein soll.

Aus erstgenannter ist eine Vorrichtung zur Wundbehandlung unter Einsatz von Unterdruck bekannt, aufweisend ein gasdichtes Wundabdeckungselement , das im am Körper des Patienten angelegten Zustand einen zwischen der jeweiligen Wunde und dem Wundabdeckungselement verbleibenden Raum bildet, und wenigstens eine Anschlussstelle, die mit dem Raum in Kontakt steht und über welche die im Raum befindliche Luft evakuiert werden kann, wobei das Wundabdeckungselement von wenigstens einem flächenhaften, die Wundsekrete aufnehmenden Wundpflegeartikel unterlegt ist, dessen Volumen im Laufe des Absorptionsprozesses zunimmt, so dass die absorbierten Wundsekrete innerhalb des Wundpflegeartikels und damit unterhalb des Wundabdeckungselementes bis zur Entfernung des Wundpflegeartikels aus dem Körper des Patienten verbleiben, der Wundpflegeartikel wenigstens eine Lage eines mit Superabsorbentien angereicherten Textilabschnittes ist, die mit einer flüssigkeitsdurchlässigen Hülle umgeben ist, und die Lage in Draufsicht auf ihre Flachseite eine Fläche hat, die 3% bis 90% kleiner als die der Hülle ist, damit sich der Wundpflegeartikel in der Nähe seiner gesamten Füllungskapazität im Querschnitt einer Kreisform annähern kann.

Aus zweitgenannter ist ein Mehrkomponentenverband zur Wundbehandlung des menschlichen oder tierischen Körpers unter Einsatz von Unterdruck bekannt, aufweisend:
ein Wundabdeckungselement zur Anbringung an Haut- und Schleimhautoberfläche, wenigstens eine Anschlussstelle, die mit dem Wundraum in Kontakt steht und über welche die im Wundraum befindlichen Stoffe evakuiert werden können, wobei dieser superabsorbierende Polymere aufweist, wobei die absorbierten Wundsekrete an Polymere gebunden im Wundraum bis zur Entfernung aus dem Wundraum verbleiben,
wobei die Polymere durch ihre Bindungskapazität wechselseitige Synergien mit den subatmosphärischen Drücken unterstützen.

Aus letztgenannter ist eine Drainagevorrichtung zur Wundbehandlung unter Einsatz von Unterdruck bekannt, aufweisend ein gasdichtes, aus folienartigem Material bestehendes Wundabdeckungselement, das im am Körper des Patienten angelegten Zustand an der Hautoberfläche um den Wundbereich herum adhäsiv befestigt ist und einen zwischen der jeweiligen Wunde und dem Wundabdeckungselement verbleibenden, abgedichteten Raum bildet, wenigstens einen Drainageschlauch, der in den Raum einsetzbar ist, über den die im Raum befindlichen Stoffe evakuiert werden können, und wenigstens einen innerhalb des Raumes angeordneten, die Wundsekrete aufsaugenden Wundpflegeartikel, der wenigstens eine Lage eines mit Superabsorbentien angereicherten Textilabschnittes aufweist, die mit einer flüssigkeitsdurchlässigen Hülle umgeben ist, wobei die absorbierten Wundsekrete innerhalb des Wundpflegeartikel und damit unterhalb des Wundabdeckungselementes bis zur Entfernung des Wundpflegeartikel aus dem Körper des Patienten verbleiben, und wobei das Wundabdeckungselement eine gasdicht verschliessbare Behandlungsöffnung aufweist, durch die der Wundpflegeartikel in den Raum einlegbar und aus dem Raum entnehmbar ist.

Der Erfindungsgemäße Wundpflegeartikel kann überdies einen an anatomische Gegebenheiten angepasste Form aufweisen. Hierzu kann er z.B. in Form einer Manschette ausgebildet sein; die über den einen Arm oder ein Bein oder ein Gelenk gestülpt werden kann, oder in Form eines an die Ferse, das Ellenbogengelenk oder dergleichen angepaßten Verbandes.

Der erfindungsgemäße Wundpflegeartikel kann außerdem so ausgebildet sein, dass er sich zur Umlage um eine chirurgisch angelegte Leitung eignet. Hierzu kann der Wundpflegeartikel z.B. wenigstens einen Schlitz aufweisen, der es ermöglicht, den Verband am Körper eines Patienten um eine Leitung (z.B. eine Drainageleitung oder einen Kathether) umzulegen, wobei dem Wundpflegeartikel ein zweiter, ebenfalls flächenhafter Wundpflegeartikel zugeordnet ist, der von dem ersten Wundpflegeartikel in einem Abstand liegt, wobei der Abstand durch einen Verbindungsstreifen oder -steg überbrückt ist. Ein solcher Wundpflegeartikel ist z.B. aus der DE202006005966 der Anmelderin der vorliegenden Erfindung bekannt, deren Inhalt vollumfänglich dem Offenbarungsgehalt der vorliegenden Beschreibung hinzugefügt werden soll.

Ebenso ist in diesem Zusammenhang bevorzugt vorgesehen, dass der Wundpflegeartikel mindestens ein Agenz aufweist, das die Blutung oder die Blutungsneigung einschränken kann.

Bei besagtem Agens kann es sich um mindestens einen chemisch und/oder physiologisch wirkenden Wirkstoff bzw. Wirkstoffkomplex oder um mindestens ein physikalisch wirkendes Wirkelement handeln. Ein solcher Wundpflegeartikel ist z.B. aus der DE 10 2007 030 931 der Anmelderin der vorliegenden Anmeldung bekannt.

### Hierzu kann der Wundpflegeartikel beispielsweise

- als im wesentlichen flacher Materialabschnitt aufweisend Absorptionsmaterial, der aus einem aufsaugenden Vlies mit darin verteilten superabsorbierenden Polymeren sowie mindestens einem chemisch und/oder physiologisch wirkende Wirkstoff bzw. Wirkstoffkomplex ausgebildet sein,
- als oder in Kombination mit einem Druck- oder Kompressionsverband,
- als eine Kombination aus einer primären, nicht oder nur unwesentlich absorbierenden Wundauflage, die mindestens einen chemisch und/oder physiologisch wirkenden Wirkstoff bzw. Wirkstoffkomplex aufweist, und einer peripher von dieser primären Wundauflage angeordneten sekundären Wundauflage, die superabsorbierende Polymere enthält, wobei ggf. zwischen beiden eine Diffusionsbarriere angeordnet ist,
- in Form eines Verbandpäckchens, aufweisend eine primäre Wundauflage mit mindestens einem chemisch und/oder physiologisch wirkenden Wirkstoffbzw. Wirkstoffkomplex sowie einem an der Wundauflage angeordneten Wickelabschnitt, der zumindest abschnittsweise superabsorbierende Polymere aufweist, und/oder
- als Materialabschnitt mit einer Längserstreckung aufweisend Absorptionsmaterial, wobei der Materialabschnitt elastisch verformbare Eigenschaften aufweist, und wobei der Materialabschnitt superabsorbierende Polymere sowie ggf. mindestens einen chemisch und/oder physiologisch wirkende Wirkstoff bzw. Wirkstoffkomplex aufweist

Bevorzugt handelt es sich bei dem chemisch und/oder physiologisch wirkenden Wirkstoff bzw. Wirkstoffkomplex um mindestens einen Stoffbzw. eine Zusammensetzung, die Blutstillende Eigenschaften aufweist. Diese Stoffe sind unter dem Oberbegriff "Hämostatika" bekannt.

Bevorzugt handelt es sich bei dem chemisch und/oder physiologisch wirkenden Wirkstoff bzw. Wirkstoffkomplex um mindestens einen Stoffbzw. eine Zusammensetzung, die Blutstillende Eigenschaften aufweist. Diese Stoffe sind unter dem Oberbegriff "Hämostatika" bekannt.

Bei dem physikalisch wirkenden Wirkelement handelt es sich z.B. um eine Abbindung, ein Druckpolster, einen Druckverband oder einen Kompressionsverband.

### Definitionen

Der Begriff "Vlies" bezeichnet ein textiles Flächengebilde aus einzelnen Fasern, das im Gegensatz zu Geweben, Gestricken und Gewirken nicht aus Garnen hergestellt wird. Vliese behalten ihre strukturelle Integrität i.d.R. durch Haftung der einzelnen Fasern aneinander. Sie werden auch als "Nonwovens" bezeichnet, und z.B. durch Walken der Fasern hergestellt.

Der Begriff "Airlaid" bezeichnet einen speziellen Vliesstoff aus Zellstoff und Polyolefinfasern, in den ggf. superabsorbierende Polymere eingebettet sind.

Der Begriff "Exsudat" bezeichnet eine über die entzündlichen Prozesse des Wundödems vom Blutplasma abgeleitete Wundflüssigkeit. So wie das Blut für den Transport von Nährstoffen und anderen Botenstoffen und damit für die Versorgung verschiedener Teile des Körpers verantwortlich ist, dient das Exsudat auf ganz ähnliche Weise der Versorgung des Wundbettes und der darin ablaufenden Heilungsprozesse. Um dieser Vielzahl an Funktionen gerecht zu werden, enthält es ein breites Spektrum an Komponenten, woraus ein spezifisches Gewicht resultiert, das leicht oberhalb dessen von Wasser liegt. Darin unterscheidet es sich auch vom Transsudat, welches von nicht-entzündlichen Prozessen abgeleitet ist und ein deutlich geringeres spezifisches Gewicht mit einem geringen Zell- und Proteingehalt aufweist. Neben der Bereitstellung von Nährstoffen für die Fibroblasten und Epithelzellen koordiniert das Exsudat die verschiedenen Prozesse der Wundheilung zeitlich und räumlich durch seinen hohen Gehalt an Wachstumsfaktoren und Zytokinen. Diese werden vor allem durch Thrombozyten, Keratinozyten, Makrophagen und Fibroblasten gebildet. Sie beeinflussen die Motilität, Migration und Proliferation der verschiedenen an der Wundheilung beteiligten Zellen. So wird das Einwandern von Zellen in den Wundgrund ebenso gefördert wie die Versorgung des neugebildeten Granulationsgewebes durch die Angiogenese. Auch die Wundreinigung wird durch das Exsudat unterstützt. Es enthält verschiedene Serin-, Cystein- und Aspartatproteasen sowie Matrix-Metalloproteasen, die in ihrer Aktivität streng reguliert irreversibel geschädigtes Gewebe abbauen und somit das Wundbett für die nachfolgenden Phasen der Heilung vorbereiten.

Bestandteile des physiologischen Exsudats sind insbesondere Salze, Glucose, Zytokine und Wachstumsfaktoren, Plasmaproteine, Proteasen (insbesondere Matrix-Metalloproteasen), Granulozyten und Makrophagen.

Unter dem Begriff "chemisch und/oder physiologisch wirkenden Wirkstoff bzw. Wirkstoffkomplex" sollen im Folgenden solche Wirkstoffe bzw. Wirkstoffkomplexe verstanden werden, die die Blutung oder die Blutungsneigung einzuschränken imstande sind, ohne dass dabei physikalische Kräfte angewendet werden müssen. Der Wirkungsweg ist hier eine chemische und/oder physiologische Interaktion mit dem Wundmilieu.

Unter dem Begriff "Wirkstoffkomplex" soll im Folgenden nicht nur ein Komplex im chemischen Sinne verstanden werden, sondern insbesondere eine Zusammensetzung synergistisch eine Wirkung hervorrufender Wirkstoffe.

Unter dem Begriff "physikalisch wirkendes Wirkelement" soll im Folgenden ein Wirkelement verstanden werden, das auf physikalischem Wege, d.h. durch die Ausübung von Druck, Zug, Kälte und dergleichen, die Blutung oder die Blutungsneigung einzuschränken imstande ist.

Unter dem Begriff "Abbindung" soll im Folgenden eine notfallmedizinische Maßnahme verstanden werden, die in der Lage ist, den arteriellen Blutfluß z.B. in einer Gliedmaße zu stoppen, um so einen unvertretbaren Blutverlust in einer Wunde zu verhindern. Indikation für eine solche Abbindung sind in der Regel traumatische Einwirkungen, die zu einer Verletzungen mindestens einer Arterie führen.

Unter dem Begriff "chronische Wunden" sollen Wunden verstanden werden, die nicht primär auf traumatische Einwirkungen zurückgehen. Zwar können traumatische Einwirkungen der ursprüngliche Auslöser einer solchen Wunde gewesen sein, aber die chronische Wunde zeichnet sich vor allem durch eine verzögerte Wundheilung auf. Chronische Wunden weisen häufig - wenn überhaupt - nur leichte Blutungen auf, dafür oftmals eine starke Exsudation.

Unter dem Begriff "leichte Blutung" soll eine Blutung verstanden werden, die nicht arteriellen Ursprungs ist, sondern ggf. venösen Ursprungs oder interstitiellen bzw. kapillaren Ursprungs, und die in jedem Fall so leicht ausfällt, dass sie nicht mittel- oder unmittelbar lebensbedrohend ist.

Unter dem Begriff "akut blutende Wunden" sollen solche Wunden verstanden werden, die zu großen Blutverlusten führen. In der Regel sind hierfür arterielle Blutungen verantwortlich, die z.B. durch traumatische Einwirkungen verursacht werden. Akut blutende Wunden können u.U. mittel- oder unmittelbar lebensbedrohend sein. Aus diesem Grunde hat bei akut blutenden Wunden die Blutungsstillung eine sehr hohe Priorität.

Unter dem Begriff "Druckverband" soll im Folgenden der aus der Notfallmedizin bekannte Druckverband verstanden werden, Dieser besteht aus einem nicht zu harten, nicht saugfähigen Gegenstand (Druckpolster) ohne scharfe oder harte Kanten, der auf eine bereits abgedeckte Wunde aufgebracht wird und mithilfe einer Wickel mit mäßigem Zug befestigt wird. Durch den ausgeübten Druck wird die Durchblutung des betreffenden Körperteils gemindert und die traumatisch geöffneten Blutgefäße werden wieder geschlossen.

Unter dem Begriff "nicht oder nur unwesentlich absorbierende Wundauflage" soll eine Wundauflage bezeichnen, die ein geringes Absorptionsvermögen für Flüssigkeiten aufweist. Insgesamt soll das Absorptionsvermögen dabei bei weniger als 60 Gew.-%, bevorzugt weniger als 20 Gew.-% des Trockengewichts der Wundauflage liegen. Primäre Aufgabe einer solchen Wundauflage ist daher auch nicht die Aufnahme von Blut oder Exsudaten, sondern die Abgabe blutstillender Agenzien im Sinne der vorliegenden Erfindung.

Unter dem Begriff "Kompressionsverband" wird hingegen in aller Regel ein Verband verstanden, der ähnlich wie ein Druckverband wirkt, jedoch auf das genannte Druckpolster verzichtet. Der Druck oder die Kompression auf die Wunde wird hierbei ausschließlich durch die Wickel ausgeübt. Hierbei kann das Wickelmaterial elastisch sein.

Alginate werden aus den Braunalgen gewonnen und zu einem faserigen Vlies verwoben Chemisch handelt es sich um Polysaccharide, und zwar Calcium- und/oder Natrimsalze der Alginsäuren. Alginate können bis zum 20fachen ihres Eigengewichtes an Flüssigkeit aufnehmen, dabei wird das Wundexsudat in die Hohlräume eingelagert. Die im Alginatgitter enthaltenen Ca²⁺ Ionen werden gegen die Na⁺ Ionen aus dem Exsudat ausgetauscht, bis der Sättigungsgrad an Na-Ionen im Alginat erreicht ist. Dabei kommt es zu einem Aufquellen der Wundauflage und zur Umwandlung der Alginatfaser in einen Gelkörper durch Aufquellen der Fasern.

Carboxymethylcellulose liegt insbesonderte in Form von Natriumcarboxymethylcellulose vor und ist unter dem Namen "Hydrofaser" im Handel. In Hygiene- und Wundprodukten werden die Fasern in eine flächige Matrix überführt. Durch die Aufnahme von Flüssigkeit aus dem Wundexsudat werden die Fasern nach und nach in ein Gelkissen umgewandelt, das die Flüssigkeit hält und nicht wieder freigibt. Dabei sind die Fasern so aufgebaut, dass das Wundexsudat nur in vertikaler Richtung aufgenommen wird. Dies bedeutet dass, solange die Kapazität reicht, das Exsudat nicht über den Wundrand fließt. Auf diese Weise kann eine Wundrandmazeration effektiv verhindert werden.

Bei dem Weichschaumstoff der Hülle kann es sich um mindestens ein Material ausgewählt aus der Gruppe enthaltend thermoplastische Weichschäume, wie Polyurethan-, Polyamid- oder Polyetherschaum, Silikonschaum sowie Cellulose-Schaum oder Naturschwamm handeln.

Naturschwämme beispielsweise der Klasse der Hornkieselschwämme (Demospongiae) weisen ähnlich wie technische Schaumstoffe ein Absorptionsvermögen für Flüssigkeiten auf. Überdies weisen sie wachstumshemmende Eigenschaften gegenüber Mikroorganismen auf, um sich vor dem Ansiedeln von sessilen Organismen zu schützen. Diese Eigenschaften können auch in Zusammenhang mit der Wundversorgung sinnvoll sein, um Bakterienwachstum in der Wundauflage und/oder in der Wunde zu verhindern. Ebenso weisen diese Schwämme wachstumshemmende Eigenschaften gegenüber Pilzen und Einzellern auf. Hinzu kommt, dass solche Schwämme Flüssigkeiten zu absorbieren imstande sind und sich daher für die Aufnahme von Exsudaten hervorragend eignen.

Besagter Naturschwamm kann in dünnen Scheiben, die z. B. durch thermisches Schneiden erzeugt worden sind, auf die Wunde aufgelegt sein.

Der Weichschaumstoff kann ggf. mehrlagig ausgestaltet sein, wobei die einzelnen Lagen bevorzugt Dicken zwischen 0,5 mm und 10 mm aufweisen können.

Der Weichschaumstoff kann offenzellig und geschlossenzellig ausgestaltet sein. Insbesondere in letzterem Falle kann vorgesehen sein, dass das Material Stanzungen und/oder Perforationen aufweist, welche den Flüssigkeitsein- und -durchtritt in bzw. durch den Weichschaumstoff beschleunigen. Überdies kann der Weichschaumstoff auch als Integralschaum ausgestaltet sein.

Einzelne Bestandteile der Hülle, insbesondere der Schaumstoffhülle, können an den Rändern der Wundauflage miteinander physikalisch verbunden sein, z.B. durch Verkleben, Vernähen oder Verschweissen. Weitere physikalische Verbindungstechniken sind hier denkbar und im Sinne der Erfindung erfasst. Als Schweißverfahren kommt insbesondere thermisches Schweißen oder Ultraschallschweissen in Frage.

### Zeichnungen und Beispiele

Die vorliegende Erfindung wird durch die im Folgenden gezeigten und diskutierten Figuren und Beispiele genauer erläutert. Dabei ist zu beachten, dass die Figuren und Beispiele nur beschreibenden Charakter haben und nicht dazu gedacht sind, die Erfindung in irgendeiner Form einzuschränken.

### Beispiel 1: Vermehrung geeigneter Phagen

Ein *Staphylococcus aureus*-Stamm wird in einem LB-Medium (Bertani, (1951), J. Bacteriol. Bd. 62, Nr. 3, S.293-300) bei 37 °C bis zu einer OD₅₇₈ von 0.6 vermehrt. Ein Konzentrat eines der Phagen ϕ11, ϕ12, ϕ20, ϕ812, ϕ131, SK311, U16 oder Twort wird in MacFarland's Lösung aufgenommen und suspendiert, anschließend wird die Staphylococcus-Kultur mit dieser Probe beimpft, und die Kultur wird weiter bei 37° C inkubiert. Ist durch die Phagenaktivitär eine OD von 0.2 erreicht, wird die Kultur durch einen Sterilfilter mit einem Porendurchmesser von 0,22 µm filtriert. Das Filtrat im Swing-Out-Rotor einer Ultrazentrifuge bei 22.000 rpm und 10° C 20 h lang zentrifugiert. Der Überstand wird abgenommen und gekühlt bzw. eingefroren. Das identische Verfahren eignet sich auch zur Vermehrung anderer geeigenter Phagen (siehe Tabelle 1 und 2). Dabei sind ggf. andere Wirtsbakterien zu verwenden.

### Beispiel 2: Reinigung und Entfernung von Endotoxinen

Um ggf. in dem in Beispiel 1 hergestellten Phagenkonzentrat enthaltene bakterielle Endotoxine zu entfernen, kann das Konzentrat ggf. einem Reinigungsschritt unterworfen werden. Hierzu kann zunächst einmal der Endotoxingehalt mit einem geeigneten Test, z.B. dem Kinetic-QCL-Test von BioWhittaker Inc., der auf einem Limulus-Amöbocyten-Lysat (LAL) und einem synthetischen Substrat basiert, bestimmt werden. Die LPS-Kontamination wird üblicherweise in Endotoxin-Units (EU) angegeben; 1 ng LPS entspricht 1-10 EU. Überschreitet der Endotoxingehalt einen kritischen Grenzwert, können die Endotoxine mit einem geeigneten Verfahren entfernt werden (z.B. Endotoxin Removal Buffer von Qiagen, Hilden).

### Beispiel 3: Herstellung einer mit einer Phagenzubereitung ausgestatteten Airlaidmatte

Das in Beispiel 1 oder 2 hergestellte Phagenkonzentrat wird mit Hilfe einer physiologischen Kochsalzlösung auf eine Konzentration von 10³ - 10²⁰ Phagen ml⁻¹ gebracht. Diese Phagenzubereitung wird mit Hilfe von Druckluft auf eine Airlaidmatte enthaltend superabsorbierende Partikel gemäß obiger Beschreibung aufgesprüht. Dabei wird pro Quadratmeter ein Volumen zwischen 1 und 100 ml aufgesprüht. Die so behandelte Airlaidmatte wird getrocknet, in Abschnitte einer Große zwischen 5 x 5 und 20 x 10 cm geschnitten und in eine Polypropylenhülle verpackt, ähnlich wie in der W003094813, der WO2007051599 und der W00152780 der Anmelderin der vorliegenden Erfindung beschrieben.

### Beispiel 4: Herstellung eines Schaumstoffs enthaltend Phagen

Das in Beispiel 2 hergestellte Phagenkonzentrat wird in einer Lyophille gefriergetrocknet und anschließend einem Polyurethan-Granulat in einem Gewichtsverhältnis von 0,01 - 1 Gew.-% beigemischt. Besagtes Gemisch wird dann gemäß Standardverfahren aus dem Stand der Technik zu einem Weichschaumstoff aufgeschäumt. Der erhaltene Weichschaumstoff wird in Scheiben mit einer Dicke von 0,5 - 10 mm geschnitten, und kann dann als Wundauflage verwendet oder mit anderen Materialien, wie sie z.B. in der noch nicht offengelegten PCT/EP2007/060304 beschrieben sind, zu einer Wundauflage verarbeitet werden. Alternativ können die gefriergetrockneten Phagen auch mittels Druck in das fertige Schaumstoffmaterial eingepresst werden.

Fig. 1: Absorption von Proteinen durch superabsorbierende Polymere

Fig. 1A zeigt in Vergrößerung einen Partikel aus dem superabsorbierenden Polymer Polyacrylat, wie es in einer Wundauflage der Anmelderin der vorliegenden Erfindung verwendet wird und z.B. in der W003094813, der WO2007051599 und der W00152780 der Anmelderin der vorliegenden Erfindung beschrieben ist. Der schwarze Balken entspricht einer Länge von 10 mm.

Besagte Partikel wurden 2 h in Rinderserum inkubiert, das eine Vielzahl von Proteinen enthält, die hier stellvertretend für bakterielle Endotoxine gelten sollen. Nach der Inkubation wurden die Partikel nach einem Standardprotokoll fixiert und mit Coomassie gefärbt. Es ist in Fig. 1A gut zu erkennen, dass der Partikel (gefärbte Flächen) Protein aufgenommen und gebunden hat. Fig. 1B zeigt als Vergleichsversuch einen entsprechenden Partikel, der in einer physiologischen Salzlösung inkubiert wurde, die keine Proteine enthielt und ansonsten identisch behandelt wurde.

Die Ergebnisse zeigen deutlich, dass eine Wundauflage enthaltend superabsorbierende Polymere in der Lage ist, die durch die Phagenaktivität freiwerdenden bakteriellen Endotoxine und Pathogenitätsfaktoren aufzunehmen und so Entzündungen, Allergien, Schocks (insbesondere anaphylaktische Schocks und/oder das Toxic Shock Syndome) und Fieber (Herxheimer-Reaktion) zu verhindern.

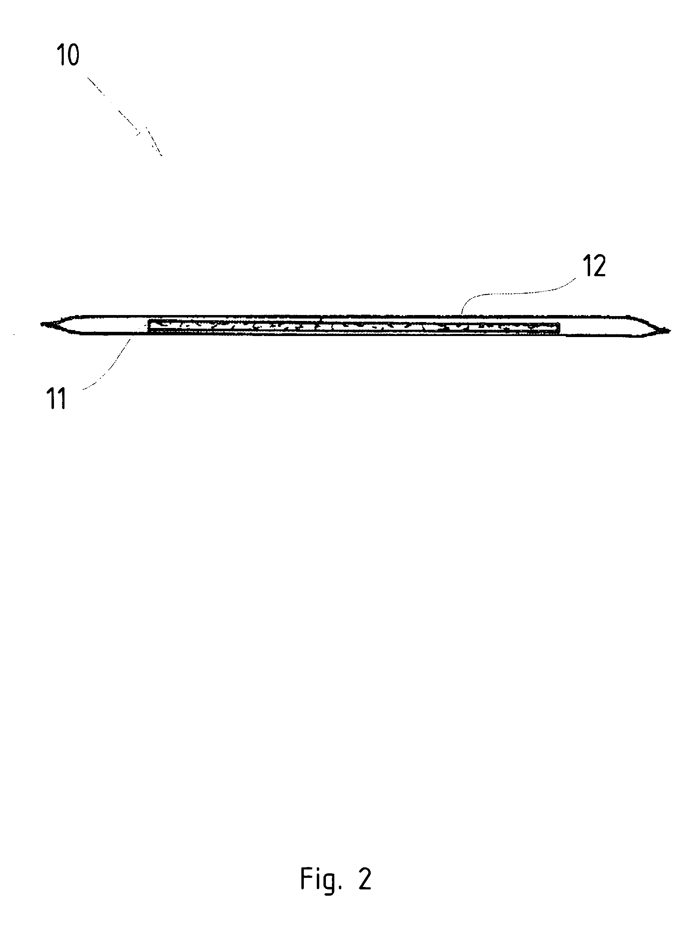

Fig. 2: Aufbau einer erfindungsgemäßen Wundauflage enthaltend Bakteriophagen

Fig. 2 zeigt beispielhaft den Aufbau einer erfindungsgemäßen Wundauflage 10 enthaltend Bakteriophagen. Die Wundauflage weist eine Matte 11 aus einem Airlaidmaterial auf, das superabsorbierende Polymere aufweist und gemäß Beispiel 3 mit einer Phagenzubereitung behandelt wurde. Außerdem weist die Wundaufglage eine Hülle 12 aus einem flüssigkeitsdurchlässigen Material auf. Insgesamt entspricht der Aufbau dieser Wundauflage im wesentlichen dem einer Wundauflage, wie sie in der W003094813, der W02007051599 und der WO0152780 der Anmelderin der vorliegenden Erfindung beschrieben ist, mit der Ausnahme, dass die Matte 11 mit einer Phagenzubereitung behandelt wurde.

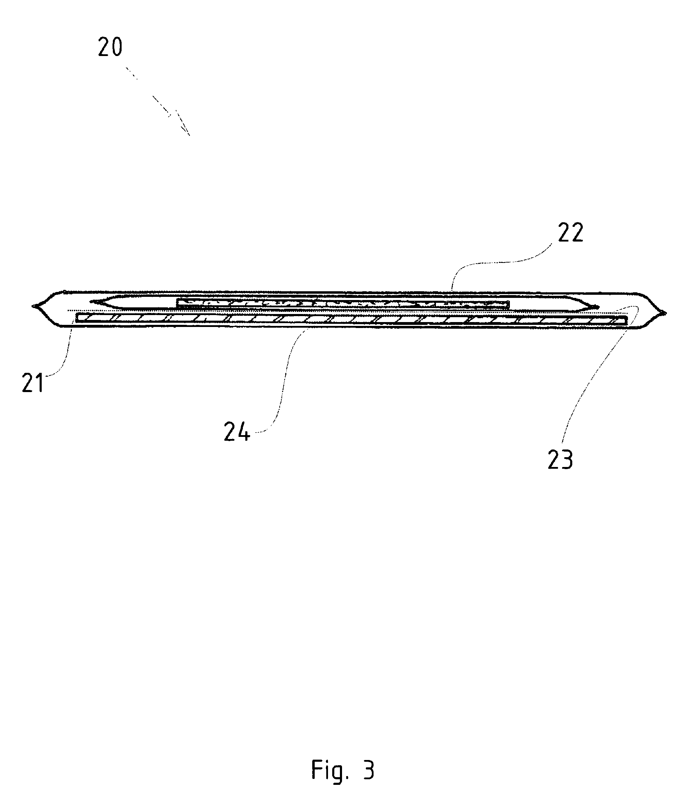

Fig. 3: Aufbau einer weiteren erfindungsgemäßen Wundauflage enthaltend Bakteriophagen

Fig. 3 zeigt beispielhaft den Aufbau einer weiteren erfindungsgemäßen Wundauflage 20 enthaltend Bakteriophagen. Diese Wundauflage entspricht im wesentlichen der in Fig. 2 gezeigten. Auf der der Wunde zugewandten Seite der Wundauflage ist eine Matte 21 aus einem nicht oder nur schwach absorbierenden Material vorgesehen, die zuvor gemäß Beispiel 3 mit einer Phagenzubereitung behandelt wurde. Auf der der Wunde abgewandten Seite der Wundauflage ist hingegen eine Matte 22 aus einem Airlaid vorgesehen, das superabsorbierende Polymere enthält. Ein solches Airlaid ist z.B. in der WO03094813, der WO2007051599 und der WO0152780 der Anmelderin der vorliegenden Erfindung beschrieben. Zwischen den beiden Matten 21 und 22 ist eine Diffusionsbarriere 23 vorgesehen, die z.B. aus Gelatine oder wasserlöslicher Stärke besteht. Außerdem weist die Wundaufglage eine Hülle 24 aus einem flüssigkeitsdurchlässigen Material auf. Ein solcher Aufbau gewährleistet, dass, wenn besagte Wundauflage auf eine Wunde aufgebracht wird, das Exsudat der Wunde zunächst in die Matte 21 eindringt, die dort vorgesehen Phagen löst und der Wunde zuführt. Dieser Prozess kann ggf. durch leichtes Anfeuchten der wundzugewandten Seite der Wundauflage beschleunigt bzw. erleichtert werden. Die zwischen beiden Matten vorgesehene Diffusionsbarriere 23 gewährleistet, dass die Wundauflage in diesem Stadium ihre saugende Aktivität noch nicht entfaltet, so dass die Phagen in der.Wunde ungestört wirksam werden können. Erst mit einer gewissen Verzögerung ("Retentionszeit"), die durch (i) die Dicke der Diffusionsbarriere, (ii) den Aufbau der Diffusionsbarriere, (iii) die Stärke der Exsudation und (iv) das Ausmaß der ggf. vorher erfolgten Anfeuchtung bedingt ist, wird die Diffusionsbarriere durchlässig, so dass der Superabsorber "anspringen" kann und die Exsudate zusammen mit den enthaltenen Phagen und den Resten der lysierten Bakterien, insbesondere der freigewordenen Endotoxine, aufnimmt. Da der lytische Zyklus der meisten Bakteriophagen in einem Zeitraum zwischen 5 min und 2 h liegt, wird die Retentionszeit durch geeignete Wahl der oben genannten vier Parameter so eingestellt, dass die Wundauflage erst nach Ablauf des lytischen Zyklus mit der Absorption beginnt.

Fig. 4: Aufnahme von Bakterien durch eine Wundauflage enthaltend superabsorbierende Polymere.

Fig. 4 zeigt die Aufnahme von Bakterien durch eine Wundauflage enthaltend superabsorbierende Polymere (Produkt "sorbion sachet" de Firma Sorbion) im Vergleich zu einer Wundauflage enthaltend einen Hydropolymerschaum. Hierzu wurden die Wundauflagen mit 50 ml einer Lösung behandelt, die 50.000 Keime der Art *Staphylococcus aureus* pro ml enthielt.

In Fig. 4A sind die an der Oberfläche der Wundauflagen nachweisbaren koloniebildenden Einheiten direkt nach der Behandlung (schwarzer Balken) bzw. 12 h nach der Behandlung (grauer Balken) dargestellt.

In Fig. 4B sind die in der Pressflüssigkeit nachweisbaren koloniebildenden Einheiten direkt nach der Behandlung (schwarzer Balken) bzw. 12 h nach der Behandlung (grauer Balken) dargestellt.

Es ist klar erkennbar, dass die Wundauflage enthaltend superabsorbierende Polymere a) die Keime sehr viel schneller aufnimmt als die Wundauflage enthaltend einen Hydropolymerschaum., und b) die Keime sehr viel besser festhält als letztere.

## Patentansprüche

1. Hygiene- oder Pflegeartikel, aufweisend
a) einen Anteil an superabsorbierenden Polymeren, und
b) eine Zubereitung aufweisend Bakteriophagen oder mindestens einen Bestandteil derselben.

2. Hygiene- oder Pflegeartikel gemäß einem der vorherigen Ansprüche, **dadurch gekennzeichnet, dass** die Bakteriophagen ausgewählt sind aus der Gruppe enthaltend native Bakteriophagen, rekombinante Bakteriophagen, deaktivierte Bakteriophagen, und/oder modifizierte Bakteriophagen.

3. Hygiene- oder Pflegeartikel gemäß einem der vorherigen Ansprüche, **dadurch gekennzeichnet, dass** der mindestens eine Bestandteil der Bakteriophagen ausgewählt sind aus der Gruppe enthaltend Lysine, Adhäsine, Endopeptidasen, Amidasen, Spikeproteine; Adhäsine, und/oder Grundplattenproteine.

4. Hygiene- oder Pflegeartikel gemäß einem der vorhergehenden Ansprüche, wobei es sich bei den superabsorbierenden Polymeren um Polymere in Pulver- oder Granulatform handelt.

5. Hygiene- oder Pflegeartikel gemäß einem der vorhergehenden Ansprüche, wobei es sich bei den Superabsorbierenden Polymeren um Polymere in Faser-, Garn-, Watte- Vlies- oder Gewebeform handelt.

6. Hygiene- oder Pflegeartikel gemäß einem der vorhergehenden Ansprüche, **dadurch gekennzeichnet, dass** die Fasern mindestens teilweise in Form eines Vlieses, eines Gewebes, eines Gewirkes, eines Airlaids und/oder eines Nonwoven vorliegen.

7. Hygiene- oder Pflegeartikel gemäß einem der vorhergehenden Ansprüche, **dadurch gekennzeichnet, dass** die verwendeten Bakteriophagen bzw. deren Bestandteile ausgewählt sind aus der Gruppe enthaltend Myoviridae und/oder T-Phagen.

8. Hygiene- oder Pflegeartikel gemäß einem der vorhergehenden Ansprüche, **dadurch gekennzeichnet, dass** es sich bei dem Hygiene- oder Pflegeartikel um einen Artikel ausgewählt aus der Gruppe enthaltend Wundpflegeartikel, Monatsbinden, Tampons, Inkontinenzartikel, Windeln, Krankenunterlagen, Stomabeutel, Stomabeuteleinlagen, Fußmatten, OP-Tücher, Redonflaschen, Taschentücher und/oder Hyperhidrose-Artikel handelt.

9. Hygiene- oder Pflegeartikel, insbesondere Wundpflegeartikel, gemäß einem der vorhergehenden Ansprüche, **dadurch gekennzeichnet, dass** der Wundpflegeartikel einen Wundexsudate absorbierenden Körper aufweist. und eine mindestens abschnittsweise um diesen Körper angeordnete Hülle aufweist.

10. Hygiene- oder Pflegeartikel, insbesondere Wundpflegeartikel, gemäß einem der vorhergehenden Ansprüche, **dadurch gekennzeichnet, dass** der Hygiene- oder Pflegeartikel, insbesondere Wundpflegeartikel weiterhin aufweist
a) einen Anschnitt aufweisend Weichschaumstoff
b) einen Abschnitt aufweisend modifizierte Cellulose, insbesondere Carboxymethylcellulose, und/oder
c) einen Abschnitt aufweisend Alginate.

11. Hygiene- oder Pflegeartikel, insbesondere Wundpflegeartikel gemäß einem der vorherigen Ansprüche, **dadurch gekennzeichnet, dass** der Artikel in Form eines Tupfers, eines Wundtuchs, einer Wundauflage, einer Wundkompresse, eines Wundpolsters, einer ggf. elastischen Wickel, einer Bandage, eines Pflasters oder eines Strumpfes vorliegt.

12. Kit für die Akut-, Notfall- oder Militärmedizinische bzw. die chronische Versorgung, aufweisend einen Wundpflegeartikel gemäß einem der vorherigen Ansprüche.

13. Verwendung von
a) superabsorbierenden Polymeren, und
b) einer Zubereitung aufweisend Bakteriophagen oder mindestens einen Bestandteils derselben
zur Herstellung eines Hygiene- oder Pflegeartikels, insbesondere eines Wundpflegeartikels, insbesondere für die Behandlung von Wunden, insbesondere chronischen Wunden.

## Claims

1. Hygiene or care article, comprising
a) an amount of superabsorbing polymers, and
b) a preparation comprising bacteriophages or at least one component of the same.

2. Hygiene or care article according to any one of the preceding claims, **characterized in that** the bacteriophages are selected from the group comprising native bacteriophages, recombinant bacteriophages, deactivated bacteriophages, and/or modified bacteriophages.

3. Hygiene or care article according to any one of the preceding claims, **characterized in that** the at least one component of the bacteriophages is selected from the group comprising lysins, adhesins, endopeptidases, amidases, spike proteins and/or base plate proteins.

4. Hygiene or care article according to any one of the preceding claims, wherein the superabsorbing polymers are polymers in form of powder or granulate.

5. Hygiene or care article according to any one of the preceding claims, wherein the superabsorbing polymers are polymers in form of fiber, yarn, cotton, non-woven or tissue.

6. Hygiene or care article according to any one of the preceding claims, **characterized in that** the fibers are at least partly present in form of a fleece, a tissue, a knitted fabric, an Airlaid and/or a Nonwoven.

7. Hygiene or care article according to any one of the preceding claims, **characterized in that** the used bacteriophages or their components are selected from the group containing Myoviridae and/or T-phages.

8. Hygiene or care article according to any one of the preceding claims, **characterized in that** the hygiene or care article is an article selected from the group comprising wound care articles, sanitary napkins, tampons, incontinence articles, diapers, protective sheets, colostomy bags, colostomy bag inserts, floor mats, surgical cloths, Redon bottles, handkerchiefs and/or hyperhidrosis articles.

9. Hygiene or care article, especially wound care article, according to any one of the preceding claims, **characterized in that** the wound care article comprises a wound exsudate absorbing body, and comprises an envelope, which is at least partially disposed around this body.

10. Hygiene or care article, especially wound care article, according to any one of the preceding claims, **characterized in that** the hygiene or care article, especially wound care article, further comprises
a) a section comprising soft foam material
b) a section comprising modified cellulose, in particular carboxymethyl cellulose, and/or
c) a section comprising alginates.

11. Hygiene or care article, especially wound care article, according to any one of the preceding claims, **characterized in that** the article is present in form of a swab, a wound cloth, a wound covering, a wound compress, a wound pad, an if necessary flexible wrap, a bandage, a plaster or a stocking.

12. Kit for acute, emergency or military-medical supply or the chronic supply, respectively, comprising a wound care article according to any one of the proceeding claims.

13. Use of
a) superabsorbing polymers, and
b) a preparation comprising bacteriophages or at least one component of the same for the manufacture of a hygiene or care article, especially a wound care article, especially for the treatment of wounds, especially chronic wounds.

## Revendications

1. Article de soin ou d'hygiène, contenant
a. une proportion de polymères super absorbants, et
b. une préparation contenant des bactériophages ou au moins un composant de ceux-ci.

2. Article de soin ou d'hygiène selon l'une des revendications précédentes, **caractérisé en ce que** les bactériophages sont choisis dans le groupe comprenant les bactériophages natifs, les bactériophages recombinants, les bactériophages désactivés, et / ou les bactériophages modifiés.

3. Article de soin ou d'hygiène selon l'une des revendications précédentes, **caractérisé en ce que** le ou les composants des bactériophages sont choisis dans le groupe comprenant la lysine, l'adhésine, les endopeptidases, les amidases, les protéines spike et/ ou les protéines de la plaque basale.

4. Article de soin ou d'hygiène selon l'une des revendications précédentes, dans lequel les polymères super absorbants sont des polymères sous forme de poudre ou de granulés.

5. Article de soin ou d'hygiène selon l'une des revendications précédentes, dans lequel les polymères super absorbants sont des polymères sous forme de fibre, de fil, de ouate, de toison ou de tissu.

6. Article de soin ou d'hygiène selon l'une des revendications précédentes, **caractérisé en ce que** les fibres sont au moins en partie présentes sous la forme d'une toison d'un tissu, d'un tricot, d'un airlaid et/ ou d'un tissu non tissé.

7. Article de soin ou d'hygiène selon l'une des revendications précédentes, **caractérisé en ce que** les bactériophages utilisés ou leurs composants sont choisis dans le groupe comprenant les Myoviridae et 1 ou les phages T.

8. Article de soin ou d'hygiène selon l'une des revendications précédentes, **caractérisé en ce qu'**il s'agit pour cet article de soin ou d'hygiène d'un article choisi dans le groupe comprenant les articles de traitement des plaies, les serviettes périodiques, les tampons, les articles contre l'incontinence, les couches, les alèses, les poches de stomie, les enveloppes pour poches de stomie, les tapis de sol, les draps chirurgicaux, les flacons de Redon, les mouchoirs et / ou les articles contre l'hyperhidrose.

9. Article de soin ou d'hygiène, notamment article de traitement des plaies, selon l'une des revendications précédentes, **caractérisé en ce que** l'article de traitement des plaies comprend un corps destiné à absorber les exsudats de la plaie, et une gaine disposée au moins par sections autour de ce corps.

10. Article de soin ou d'hygiène, notamment article de traitement des plaies, selon l'une des revendications précédentes, **caractérisé en ce que** l'article de soin ou d'hygiène, notamment l'article de traitement des plaies, présente en outre :
a. une section comprenant de la mousse souple
b. une section comprenant de la cellulose modifiée, notamment de la carboxyméthylcellulose, et 1 ou
c. une section comprenant des alginates.

11. Article de soin ou d'hygiène, notamment article de traitement des plaies, selon l'une des revendications précédentes, **caractérisé en ce que** l'article se présente sous la forme d'un tampon, d'une gaze, d'un pansement, d'une compresse, d'un coussinet rembourré, le cas échéant d'un enveloppement élastique, d'un bandage, d'un sparadrap ou d'une chaussette.

12. Kit de prise en charge médicale aiguë, d'urgence ou militaire ou bien chronique, comprenant un article de traitement des plaies selon l'une des revendications précédentes.

13. Utilisation :
a. de polymères super absorbants, et
b. d'une préparation contenant des bactériophages ou au moins un composant de ceux-ci
pour la fabrication d'un article d'hygiène ou de soin, notamment d'un article de traitement des plaies, en particulier pour le traitement des plaies, et notamment des plaies chroniques.
